# EUROPÄISCHE PATENTSCHRIFT

(11) **EP 3 359 945 B1**
(45) Veröffentlichungstag und Bekanntmachung des Hinweises auf die Patenterteilung: **26.03.2025**
(21) Anmeldenummer: 16777687.1
(22) Anmeldetag: 06.10.2016
(51) Int. Cl.: G01M 99/00, B05B 15/00, A61M 15/00

(54) **PRÜFSYSTEM UND PRÜFVERFAHREN**
TEST SYSTEM AND TEST METHOD
SYSTEME DE CONTROLE ET PROCEDE DE CONTROLE

(30) Priorität: 09.10.2015 EP 15189068; 12.05.2016 EP 16169471
(43) Veröffentlichungstag der Anmeldung: 15.08.2018
(73) Patentinhaber: Boehringer Ingelheim Microparts GmbH, 44227 Dortmund (DE)
(72) Erfinder: EICHER, Joachim, 55216 Ingelheim am Rhein (DE); EIGEMANN, Jutta, 55216 Ingelheim am Rhein (DE); GESER, Johannes, 55216 Ingelheim am Rhein (DE); PETERS, Andreas, 55216 Ingelheim am Rhein (DE); KOELBEL, Hans-Juergen, 55216 Ingelheim am Rhein (DE)
(74) Vertreter: Lutze, Oliver
(86) Internationale Anmeldenummer: PCT/EP2016/073834
(87) Internationale Veröffentlichungsnummer: WO 2017/060328

(56) Entgegenhaltungen:
- EP-A1- 1 613 437
- EP-A1- 2 381 237
- EP-A1- 2 381 237
- EP-B1- 1 613 437
- US-A1- 2004 231 667
- US-A1- 2004 231 667

## Beschreibung

Die vorliegende Erfindung betrifft ein System bzw. Prüfsystem zum Prüfen der Funktionsfähigkeit eines Zerstäubers zur Abgabe eines Fluids als Aerosol gemäß dem Oberbegriff des Anspruchs 1 sowie ein Verfahren bzw. Prüfverfahren zum Prüfen der Funktionsfähigkeit eines Zerstäubers zur Abgabe eines Fluids als Aerosol gemäß dem Oberbegriff des Anspruchs 10.

Unter dem Begriff "Zerstäuber" ist vorzugsweise eine konstruktive Einrichtung zu verstehen, die insbesondere dazu ausgebildet ist, ein Fluid, insbesondere eine Arzneimittelformulierung, zu zerstäuben bzw. in ein Aerosol zu überführen. Besonders bevorzugt ist ein Zerstäuber im Sinne der vorliegenden Erfindung ein Inhalator zur Inhalation eines Fluids als Aerosol. Vorzugsweise weist ein Zerstäuber im Sinne der vorliegenden Erfindung einen Behälter als Reservoir für ein zu zerstäubendes Fluid auf. Vorzugsweise ist der Behälter in den Zerstäuber einsetzbar oder (zum Beispiel durch Öffnen einer Kappe o.ä.) zugänglich in diesem angeordnet bzw. kann ausgewechselt werden. Insbesondere wird an dieser Stelle ein Zerstäuber betrachtet, bei dem der Behälter zur Abgabe des Fluids relativ zum Gehäuse oder Gehäuseteil - vorzugsweise axial - bewegbar ist. Ein solcher Zerstäuber ist beispielsweise in der WO 09/047173 A2 offenbart.

Bei der Zerstäubung eines Fluids, insbesondere einer flüssigen Arzneimittelformulierung, mittels eines Zerstäubers der eingangs erwähnten Art soll eine möglichst genau definierte Menge an Wirkstoff in ein Aerosol zur Inhalation überführt werden. Das auf diese Weise erzeugte Aerosol soll sich durch einen kleinen Mittelwert der Tropfengröße bei einer schmalen Tropfengrößenverteilung und einen niedrigen Impuls bzw. eine niedrige Ausbreitungsgeschwindigkeit auszeichnen. Vorzugsweise weisen die so erzeugten Tropfen einen Tropfendurchmesser von weniger als 5 µm, insbesondere zwischen 2µm und 5 µm, auf, da Tropfen dieser Größe bei der Inhalation in geeigneter Weise in Lungensystemen abgeschieden werden.

Der Begriff "Fluid" ist im Sinne der vorliegenden Erfindung breit zu verstehen und auszulegen. Insbesondere umfasst der Begriff "Fluid" neben flüssigen Lösungen auch Dispersionen, Suspensionen o. dgl.

Unter dem Begriff "Aerosol" ist bei der vorliegenden Erfindung vorzugsweise eine wolkenartige oder nebelartige Ansammlung einer Vielzahl von Tropfen eines vorzugsweise mittels eines Zerstäubers zerstäubten Fluids zu verstehen, vorzugsweise wobei die Tropfen eine geringe Geschwindigkeit und/oder zumindest im Wesentlichen ungerichtete Bewegungsrichtungen aufweisen. Ein "Aerosol" kann beispielsweise eine kegelförmige Tropfenwolke aufweisen oder bilden, insbesondere wobei die Hauptausbreitungsrichtung der Tropfenwolke zumindest im Wesentlichen der Hauptaustrittsrichtung bzw. Austrittsimpulsrichtung entspricht.

Bei der Herstellung bzw. Fertigung von Zerstäubern der eingangs erwähnten Art ist es erforderlich, die Zerstäuber - beispielsweise im Rahmen des Qualitätsmanagements - auf Fehler bzw. hinsichtlich ihrer Funktionsfähigkeit zu untersuchen und/oder fehlerhafte oder fehleranfällige Zerstäuber auszusortieren, beispielsweise bei einer Vollprüfung bzw. 100%-Prüfung und/oder im Rahmen von Stichprobenprüfungen.

Unter dem Begriff "Funktionsfähigkeit" ist vorzugsweise diejenige Fähigkeit des Zerstäubers zu verstehen, die zur Abgabe bzw. Zerstäubung des Fluids mittels des Zerstäubers bzw. zur Erfüllung des vorgesehenen Zwecks bzw. der vorgesehenen Funktion notwendig ist. Vorzugsweise entspricht die Funktionsfähigkeit des Zerstäubers dem Grad, inwieweit Anforderungen an den Zerstäuber bzw. an die (Soll-) Funktion(en) des Zerstäubers erfüllt werden können.

Vorzugsweise wird die Funktionsfähigkeit anhand von Messwerten geprüft, die während einer Betätigung des Zerstäubers ermittelt werden. Besonders bevorzugt werden - beispielsweise im Rahmen eines Qualitätssystems - Sollwerte und/oder Grenzwerte festgelegt, innerhalb derer die Messwerte eines funktionsfähigen Zerstäubers liegen dürfen.

In erster Linie sollte ein funktionsfähiger Zerstäuber für die Erzeugung eines inhalierbaren Aerosols eine definierte Menge des Fluids bzw. der Arzneimittelformulierung in Form einer Aerosolwolke bzw. eines Sprühnebels mit definierten Eigenschaften abgeben. Diese Grundfunktionsfähigkeit des Zerstäubers kann anhand von Messungen der erzeugten Aerosolwolke bzw. des erzeugten Sprühnebels überprüft werden, beispielsweise mittels direkter Messungen von Tröpfchengrößenverteilungen (z.B. mittels optischer Verfahren oder Kaskadenimpaktoren), Messungen der im Sprühnebel insgesamt abgegebenen Masse des Fluids etc.

Bei manchen Zerstäubern wie beispielsweise bei Nasenspray-Pumpen können die Eigenschaften des Sprühnebels wie Sprühbild, Geometrie der Sprühwolke oder Tröpfchengrößenverteilung von der Art und Weise abhängen, wie der Zerstäuber bzw. die Pumpe ausgelöst wird. Bei der typischen Nasenspray-Pumpe wird zur Auslösung ein die Auslassöffnung enthaltendes Oberteil gegen eine Rückstellkraft in Richtung eines Medikamentenbehälters gedrückt. Eine langsame Betätigung würde dabei wahrscheinlich zu einen Flüssigkeitsausfluss mit nur geringer Vernebelung führen, und eine sehr schnelle Betätigung würde eventuell einen so feinen Sprühnebel erzeugen, dass ein großer Teil der Tröpfchen in tiefere Atemwege inhaliert wird anstatt an der Nasen-Schleimhaut adsorbiert zu werden. Damit die Funktionsfähigkeit einer solchen Nasenspray-Pumpe ohne diesen individuellen Einfluss des Anwenders innerhalb von Kriterien überprüft werden kann, wie sie im Rahmen einer klinischen Zulassung festgelegt werden, wird in der WO2004/091806A1 bzw. EP 1613437 A1 die Verwendung einer Vorrichtung vorgeschlagen, in der eine solche Nasenspray-Pumpe oder auch ein sogenanntes MDI ("metered dose inhaler") maschinell gesteuert ausgelöst werden können. Durch die Einstellbarkeit dieser Ansteuerung ermöglicht die Vorrichtung in Kombination mit Messungen am Zerstäuber die Untersuchung der Zusammenhänge zwischen dem mechanischen Zusammenspiel der beweglichen und unbeweglichen Zerstäuberteile und der Funktionsweise des Zerstäubers. Als Messverfahren wird hierzu beispielsweise die Auswertung von Sprühbildern auf dünnlagigen Chromatografie-Platten (TLC) beschrieben. Auf diese Weise können Daten erzeugt werden, mit deren Hilfe Parameter für automatische Auslösesysteme bestimmt werden können, die in Routineprüfungen (verglichen mit vom Prüfer manuell durchgeführte Auslösevorgängen) Schwankungen reduzieren.

Die US 2004/0231667 A1 offenbart einen Medikamenten Dispenser, der ähnlich einem MDI einen Medikamenten Behälter mit einem Ventil-Abgabemechanismus umfasst Durch ein Antriebsmittel wird eine Relativbewegung des Behältersitzes zur Auslösung des Abgabemechanismus vorangetrieben. Zusätzlich wird eine Testapparatur vorgestellt, um im Labor die Funktion des Ventil-Abgabemechanismus zu überprüfen. Hierin wird der Behältersitz angetrieben durch einen elektrischen DC-Motor linear verschoben und die Aspekte des Abgabeprozesses werden mittels Sensoren gemonitort.

Aus der DE 10136554 A1 ist ein Laborverfahren zur Bestimmung der Partikelgrößenverteilung in einem Aerosol aus einem Inhalator und eine Vorrichtung zur Durchführung des Verfahrens bekannt Darin wird vorgeschlagen, die Partikelgröße von Aerosolpartikeln mittels Laserdiffraktometrie bzw. einer Laserbeugungsmethode zu messen. Hierbei werden Messbedingungen geschaffen, welche die Bedingungen bei einer bestimmungsgemäßen Anwendung des Inhalators durch einen Patienten, d.h. insbesondere die hohe Luftfeuchte im menschlichen Mund-Rachen-Bereich, realitätsnah nachstellen. Aus der EP 2381237 A1 ist ein darauf aufbauendes Labormessverfahren bekannt, bei dem die Ermittlung des lungengängigen Aersolanteils (Feinpartikelanteil) mit einer Sprayzeitmessung kombiniert wird. Bei der Partikelmessung wird hierbei gestreutes Laserlicht durch eine Sammellinse zu einem Halbleiter-detektor hin gebündelt und mittels Auswerteverfahren basierend auf der Theorie der Mie-Streuung oder der Fraunhofer Methode ausgewertet

Weitere mögliche Messgrößen sind beispielsweise die Durchflussgeschwindigkeit in den Düsenkanälen des Zerstäubers, das abgegebenen Volumen und/oder die abgegebenen Masse des Fluids, insbesondere pro Auslösung und/oder pro Zeiteinheit, das Leckagevolumen und/oder die Leckagemasse des Fluids im Zerstäuber und/oder der Druck und/oder die Druckverluste des Fluids im Zerstäuber. Direkte Messungen solcher Messgrößen sind unter Umständen aufwändig und daher nur bedingt in Serienproduktionsprozessen, wie insbesondere einer automatisierten Prüfung und/oder einer 100%-Prüfung oder 100%-Sortierprüfung, durchführbar.

Vor diesem Hintergrund liegt der vorliegenden Erfindung die Aufgabe zugrunde, ein Prüfsystem sowie ein Prüfverfahren zum Prüfen der Funktionsfähigkeit eines Zerstäubers anzugeben, vorzugsweise wobei etwaige Fehler des Zerstäubers einfach, kostengünstig, schnell und/oder zuverlässig identifizierbar, bestimmbar, abschätzbar und/oder indizierbar sind bzw. identifiziert, bestimmt, abgeschätzt und/oder indiziert werden.

Insbesondere sollte sich das Prüfverfahren bzw. das Prüfsystem für den Einsatz in einem Serienproduktionsprozess eignen.

Die obige Aufgabe wird durch ein Prüfsystem gemäß Anspruch 1 oder ein Prüfverfahren gemäß Anspruch 10 gelöst. Vorteilhafte Weiterbildungen sind Gegenstand der Unteransprüche.

Die erfindungsgemäße Prüfvorrichtung bzw. das erfindungsgemäße System zum Prüfen der Funktionsfähigkeit eines Zerstäubers weist vorzugsweise einen Zerstäuber sowie eine Prüfvorrichtung zum Prüfen des Zerstäubers auf.

Ein Aspekt der vorliegenden Erfindung liegt darin, dass in einer Funktionsprüfung - zumindest teilweise - die Funktionsfähigkeit des Zerstäubers anhand von Messungen am Zerstäuber selbst, vorzugsweise an einem oder mehreren beweglichen Teilen des Zerstäubers (insbesondere an Teilen, die während der Betätigung bewegt werden), geprüft wird. Vorzugsweise werden hierbei Messwerte für Messgrößen ermittelt, für die insbesondere aufgrund des Funktionsprinzips des entsprechenden Zerstäubers eine Korrelation zu Eigenschaften des Sprühnebels besteht. Hierdurch kann eine Vorrichtung zur Überprüfung der Funktionsfähigkeit des Zerstäubers bzw. ein Prüfsystem bereitgestellt werden, in dem die Messzeiten allenfalls geringfügig länger als die für die Betätigung des Zerstäubers notwendigen Zeiten sind. Bevorzugt findet diese Funktionsprüfung in einer automatisierten 100%-Prüfungen oder 100%-Sortierprüfungen statt.

Erfindungsgemäß wird bei einem Zerstäuber, bei dem sich ein Behälter mit dem abzugebenden Fluid während der Abgabe des Fluids bewegt, die Bewegung des Behälters gemessen und die mittels der zugehörigen Messeinrichtung ermittelten Messwerte werden mit Soll- und/oder Grenzwerten in einer Datenverarbeitungseinrichtung (z.B. Computer-unterstützt) verglichen. Entsprechen die Messwerte nicht den (insbesondere mit Abweichungsintervallen definierten) Sollwerten und/oder liegen die Messwerte außerhalb eines durch die Grenzwerte definierten Bereichs, so wird der Zerstäuber im bzw. vom Prüfsystem als fehlerhaft identifiziert und automatisch aussortiert. Die Durchlaufzeit eines Zerstäubers durch ein derartiges automatisiertes System ist bei geeigneter elektronischer Auslegung im Wesentlichen bestimmt durch die Dauer der tatsächlichen Messung - die erfindungsgemäß nach der vollständigen Abgabe des Fluids / Sprühstoßes abgeschlossen ist.

Das Prüfverfahren ist daher gut geeignet für den Einsatz in einer Serienproduktion und auch insbesondere geeignet für den Einsatz in einer 100%-Prüfung.

Gemäß einem weiteren Aspekt der vorliegenden Erfindung werden in dem Prüfsystem solche Messungen am Zerstäuber selbst mit Messungen an der von ihm erzeugten Aerosolwolke bzw. dem von ihm erzeugten Sprühnebel kombiniert.

Bevorzugt weist das Prüfsystem hierzu eine Sprayparametermesseinrichtung und/oder eine bildgebende Aufnahmeeinrichtung auf, die mit der Datenverarbeitungseinrichtung des Systems verbunden ist. In der Sprayparametermesseinrichtung werden vordefinierte Sprayparameter anhand von Sprühlichtbildern gemessen, wobei die Messwerte mittels der Datenverarbeitungseinrichtung mit vordefinierten Grenzwerten verglichen werden. Alternativ oder zusätzlich werden Bilder aus einer bildgebenden Aufnahmeeinrichtung in der Datenverarbeitungseinrichtung mit Referenzbildern verglichen.

Ein Zerstäuber wird automatisch als fehlerhaft identifiziert (und insbesondere aussortiert), wenn die für ihn ermittelten Messwerte bzw. Bilder nicht mit den Sollwerten bzw. Referenzbildern entsprechen und/oder außerhalb eines Bereichs liegen, der durch die Grenzwerte bzw. Referenzbilder definiert ist.

Besonders bevorzugt werden in der Sprayparametermesseinrichtung Lichtschnitte oder Lichtvorhänge zur Abbildung der Sprühlichtbilder erzeugt.

Bevorzugt beinhaltet die Fertigung des Zerstäubers neben den Schritten zur Montage des Zerstäubers sowohl ein automatisiertes Sortier-Prüfverfahren, in dem ausgewählte Messgrößen gemessen werden, welche die generelle Funktionsfähigkeit des Zerstäubers wieder spiegeln, als auch eine Stichprobenziehung, wobei die stichprobenartig ausgewählten Zerstäuber einer detaillierten Laborprüfung unterzogen werden. In so einer Laborprüfung werden die ausgewählten Zerstäuber vorzugsweise zerstörend getestet, wobei ein Verwendungszyklus durch den Anwender nachgestellt wird. Die Laborprüfung beinhaltet dabei auch die Durchführung von (im Vergleich zur Sortierprüfung) aufwendigeren Messungen wie insbesondere Messungen der Tröpfchengrößenverteilung wie beispielsweise aus der DE 101 36 554 A1 bekannt und/oder Messungen der Masse des ausgebrachten Fluids.

Der Zerstäuber weist vorzugsweise eine Möglichkeit zum Einsetzen und/oder Auswechseln eines Behälters mit einem Fluid auf (eine solche Möglichkeit kann beispielsweise durch eine Zugangsöffnung im Gehäuse des Zerstäubers und/oder durch ein abnehmbares oder öffnenbares Gehäuseteil gegeben sein). Vorzugsweise benutzt ein Anwender den Zerstäuber mit einem insbesondere einsetzbaren und besonders bevorzugt auswechselbaren Behälter mit einem Fluid, wobei der Behälter zur Abgabe bzw. Zerstäubung des Fluids bzw. Bildung eines Aerosols relativ zum Gehäuseteil bewegbar ist.

Bei den Laborprüfungen, bei denen ein Verwendungszyklus durch einen Anwender nachgestellt wird, werden vorzugsweise Behälter verwendet, die mit dem Fluid gefüllt sind, das der Anwender zerstäuben möchte: Im Falle der Prüfung von Inhalatoren sind die Behälter also bevorzugt mit einer flüssigen Arzneimittelformulierung oder mit einem geeigneten Placebo befüllte Kartuschen.

Bei der 100%-Prüfungen oder 100%-Sortierprüfungen wird der Zerstäuber hingegen bevorzugt mit eingesetzten Behältern getestet, welche eine Flüssigkeit oder Prüfflüssigkeit enthalten, die sich insbesondere rückstandslos aus dem Zerstäuber entfernen lässt (zur Vermeidung von Kontaminationen oder Aufkonzentration). Die entsprechende Flüssigkeit wie beispielsweise Wasser oder besonders bevorzugt Ethanol sollte einen sehr hohen Reinheitsgrad aufweisen. Vorzugsweise weist der Zerstäuber bei der 100%-Prüfungen oder 100%-Sortierprüfungen einen Behälter auf, der mit der verwendeten Prüfvorrichtung wechselwirken kann. Insbesondere weist der Behälter hierzu z.B. im Fall von optischen Messungen eine geeignete Reflexionsoberfläche auf (oder im Fall von elektrischen oder induktiven Messungen eine geeignete Leitfähigkeit oder geeignete magnetische Eigenschaften oder im Fall von taktilen oder mechanischen Messungen geeignete Konturen etc.). Optional weist der Behälter eine auslesbare Kennzeichnung auf.

Bevorzugt basiert der Zerstäuber auf einem aktiven Zerstäubungsprinzip, insbesondere wird für die Zerstäubung notwendige Energie aus einem Energiespeicher freigesetzt und/oder insbesondere läuft die Zerstäubung nach Betätigung einer Auslösetaste, eines Schalters oder desgleichen am Zerstäuber automatisch ab.

Besonders bevorzugt weist der Zerstäuber zur Druckerzeugung für die Zerstäubung des Fluids einen mechanischen Pumpmechanismus, insbesondere einen Kolbenpumpmechanismus, auf, vorzugsweise wobei der Behälter mit einem beweglichen Teil des Pumpmechanismus mitbewegt wird. Vorzugsweise wird der Pumpenmechanismus mit einer Feder als Energiespeicher kombiniert.

Ein weiterer, auch unabhängig realisierbarer Aspekt der vorliegenden Erfindung liegt darin, dass bei der 100%-Prüfung oder 100%-Sortierprüfung das System bzw. die Prüfvorrichtung eine Messeinrichtung zum - vorzugsweise optischen, mechanischen und/oder elektrischen und/oder berührungslosen - Messen bzw. zur messtechnischen Erfassung der Bewegung des Behälters bzw. des Behälterbodens des Zerstäubers bei bzw. während der Zerstäubung bzw. Abgabe des Fluids aufweist, vorzugsweise um die Funktionsfähigkeit des Zerstäubers zu prüfen.

Besonders bevorzugt ist die Messeinrichtung zum - vorzugsweise optischen, mechanischen und/oder elektrischen und/oder berührungslosen - Messen der Geschwindigkeit des Behälters bzw. des Behälterbodens, des (axialen) Hubs des Behälters bzw. des Behälterbodens und/oder der Zeit des Hubs ausgebildet. Auf diese Weise wird ein besonders einfaches, schnelles, automatisierbares und/oder in einen Fertigungsprozess integrierbares Prüfsystem bereitgestellt.

Unter dem Begriff "Hub" ist hierbei die Strecke bzw. der Durchlauf über eine Strecke zwischen zwei Endlagen zu verstehen, zwischen denen sich der Behälter bzw. der Behälterboden insbesondere zwischen den Situationen vor und nach Auslösen des Zerstäubers bewegt.

Unter dem Begriff "Bewegung" ist vorzugsweise die Geschwindigkeit des Behälters bzw. des Behälterbodens, der Hub des Behälters bzw. des Behälterbodens und/oder die Zeit des Hubs des Behälters bzw. des Behälterbodens bei bzw. während der Abgabe bzw. Zerstäubung des Fluids zu verstehen. Insbesondere umfasst die Bewegung des Behälters bzw. des Behälterbodens die Position des Behälters bzw. des Behälterbodens in Abhängigkeit von der Zeit bei der Abgabe bzw. Zerstäubung des Fluids und/oder ist die Bewegung durch die Position des Behälters bzw. des Behälterbodens als Funktion der Zeit messbar, bestimmbar und/oder quantifizierbar. Ganz besonders bevorzugt ist das Prüfsystem bzw. die Messeinrichtung dazu ausgebildet, die Bewegung, insbesondere die Geschwindigkeit, den Hub und/oder die Zeit des Hubs, des Behälters bzw. des Behälterbodens bei der Abgabe des Fluids zu messen bzw. zu quantifizieren, insbesondere mittels entsprechender Messwerte.

Vorzugsweise können für einen funktionsfähigen bzw. zumindest im Wesentlichen fehlerfreien Zerstäuber vordefinierte Sollwerte für die Behälterbewegung festgelegt werden. Messgrößen sind hierbei vorzugsweise die Behältergeschwindigkeit, der Behälterhub (d.h. der Bewegungsweg des Behälters) und/oder die Zeit der einem Behälterhub entsprechenden Behälterbewegung. Vorzugsweise können diese Messgrößen oder das Zusammenspiel dieser Messgrößen mit Eigenschaften wie der Durchflussgeschwindigkeit in den Düsenkanälen des Zerstäubers, dem abgegebenen Volumen und/oder der abgegebenen Masse des Aerosols, insbesondere pro Auslösung und/oder pro Zeiteinheit, dem Leckagevolumen und/oder der Leckagemasse des Fluids im Zerstäuber und/oder dem Druck und/oder den Druckverlusten des Fluids im Zerstäuber korreliert werden.

Vorzugsweise werden für die Messgrößen eines funktionsfähigen bzw. zumindest im Wesentlichen fehlerfreien Zerstäubers Sollbereiche und/oder Grenzwerte definiert, vorzugsweise wobei die Sollwerte, Sollbereiche bzw. Grenzwerte konzeptionell, empirisch, numerisch, praktisch und/oder theoretisch ermittelt worden sind.

Die vorliegende Erfindung betrifft besonders bevorzugt die Prüfung eines Zerstäubers mit einer mechanischen bzw. manuell betätigbaren Pumpe bzw. einem mechanischen bzw. manuell betätigbaren Spannmechanismus, um das Fluid unter Druck zu setzen bzw. das Aerosol zu bilden. Hierbei kann vorgesehen sein, dass eine Bewegung des Behälters zu einer Kolbenbewegung einer Pumpe bzw. eines Druckerzeugers bzw. Ventils des Zerstäubers und/oder eine Bewegungsgeschwindigkeit des Behälters zu einer Abgabegeschwindigkeit des Fluids korrespondiert Es sind jedoch grundsätzlich auch andere Mechanismen möglich, insbesondere bei denen die Abgabemenge, die Abgabegeschwindigkeit und/oder die Eigenschaften des Aerosols zu Charakteristika der Behälterbewegung korrespondieren. Es hat sich als besonders vorteilhaft erwiesen, die Funktionsfähigkeit bzw. Einhaltung von Sollwerten oder Toleranzbereichen zumindest auch anhand der Behälterbewegung während der Abgabe des Fluid zu überprüfen.

Das vorschlagsgemäße Verfahren bzw. Prüfverfahren zum Prüfen der Funktionsfähigkeit des Zerstäubers zur Abgabe eines Fluids als Aerosol zeichnet sich dadurch aus, dass die Bewegung des Behälters des Zerstäubers bei der Abgabe bzw. Zerstäubung des Fluids - vorzugsweise mittels einer Messeinrichtung - gemessen und/oder - vorzugsweise mittels einer Datenverarbeitungseinrichtung - ausgewertet wird.

Besonders bevorzugt wird die Geschwindigkeit des Behälters bzw. des Behälterbodens, der Hub des Behälters bzw. des Behälterbodens und/oder die Zeit des Behälterhubs bei der Abgabe des Fluids oder einer Dosis des Fluids gemessen und/oder ausgewertet und/oder mit Sollwerten, insbesondere einer Sollgeschwindigkeit, einem Sollhub oder einer Sollzeit, und/oder Grenzwerten, insbesondere einer Maximal- und/oder Minimalgeschwindigkeit, einem Maximal- und/oder Minimalhub und/oder einer Maximal- und/oder Minimalzeit, verglichen. Auf diese Weise werden entsprechende Vorteile erreicht.

Besonders bevorzugt wird von der Messeinrichtung die Bewegung des Behälters mittels optischer Triangulation gemessen. Vorzugsweise wird dabei ein sichtbarer modulierter Lichtpunkt auf die Oberfläche des Behälterbodens projiziert und eine Reflexion dieses Lichtpunktes zumindest teilweise von einer Empfängeroptik, die insbesondere in einem definierten Winkel angeordnet ist, abstandsabhängig auf einem (in der Empfängeroptik beinhalteten) ortsauflösenden Element (z.B. ein CCD-Element) bzw. Sensor abgebildet. Bevorzugt wird von einem digitalen Signalprozessor der Abstand des Behälterbodens aus dem Ausgangssignal des Sensors berechnet.

Erfindungsgemäß sind die Messeinrichtung und eine zugehörige Datenverarbeitungseinrichtung Teil einer automatisierten Prüfvorrichtung, in der diejenigen Zerstäuber automatisch als fehlerhafte Zerstäuber aussortiert werden, für welche die ermittelten Messwerte außerhalb der vordefinierten Grenzwerte liegen. Die Datenverarbeitungseinrichtung ist also dazu ausgebildet, von der Messeinrichtung ermittelte Messwerte auszuwerten und mit Sollwerten und/oder Grenzwerten zu vergleichen und den Zerstäuber im Prüfsystem als fehlerhaft zu identifizieren, wenn die für ihn ermittelten Messwerte nicht den Sollwerten entsprechen und/oder außerhalb eines Bereichs liegen, der durch die Grenzwerte bestimmt ist. Für die Aussortierung eines als fehlerhaft identifizierten Zerstäubers weist das erfindungsgemäße System eine Ausgabeeinrichtung auf, in welche der Zerstäuber nach Identifizierung als fehlerhaft verschoben wird und/oder aussortiert wird.

Ein weiterer, auch unabhängig realisierbarer Aspekt der vorliegenden Erfindung betrifft eine Prüfvorrichtung zum Prüfen der Funktionsfähigkeit eines Zerstäubers, wobei der Zerstäuber zur Abgabe eines Fluids als Aerosol ausgebildet ist, wobei die Prüfvorrichtung eine Halteeinrichtung zum Halten bzw. Einspannen des Zerstäubers, eine Betätigungseinrichtung zum Betätigen des Zerstäubers, eine Messeinrichtung und eine Datenverarbeitungseinrichtung aufweist, wobei die Messeinrichtung dazu ausgebildet ist, ein sich bei der Abgabe des Fluids bewegenden Teil des Zerstäubers zu messen, und wobei die Datenverarbeitungseinrichtung dazu ausgebildet ist, von der Messeinrichtung ermittelte Messwerte auszuwerten und mit Sollwerten und/oder Grenzwerten zu vergleichen und den Zerstäuber automatisch als fehlerhaft zu identifizieren, wenn die ermittelten Messwerte nicht den Sollwerten entsprechen und/oder außerhalb eines Bereichs liegen, der durch die Grenzwerte bestimmt ist.

Gemäß einem weiteren, auch unabhängig realisierbaren Aspekt der vorliegenden Erfindung wird eine Prüfvorrichtung zum Prüfen der Funktionsfähigkeit eines Zerstäubers verwendet, wobei der Zerstäuber zur Abgabe eines Fluids als Aerosol ausgebildet ist und einen vorzugsweise einsetzbaren und besonders bevorzugt auswechselbaren Behälter mit dem Fluid und ein Gehäuseteil aufweist, wobei der Behälter zur Abgabe des Fluids relativ zum Gehäuseteil bewegt wird und die Bewegung des Behälters bei der Abgabe des Fluids gemessen wird. Auf diese Weise werden entsprechende Vorteile realisiert.

Weitere Aspekte, Merkmale, Eigenschaften und Vorteile der vorliegenden Erfindung ergeben sich aus den Ansprüchen der folgenden Beschreibung einer bevorzugten Ausführungsform anhand der Zeichnungen. Es zeigt:
- Fig. 1: einen schematischen Schnitt eines Zerstäubers im ungespannten Zustand;
- Fig. 2: einen schematischen, um 90° Grad gegenüber Fig. 1 gedrehten Schnitt des Zerstäubers im gespannten Zustand;
- Fig. 3: eine schematische Ansicht eines vorschlagsgemäßen Prüfsystems mit dem Zerstäuber gemäß Fig. 1 und einer vorschlagsgemäßen Prüfvorrichtung; und
- Fig. 4: einen schematischen Verlauf des Behälterhubs in Abhängigkeit von der Zeit.

In den teilweise nicht maßstabsgerechten, nur schematischen Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

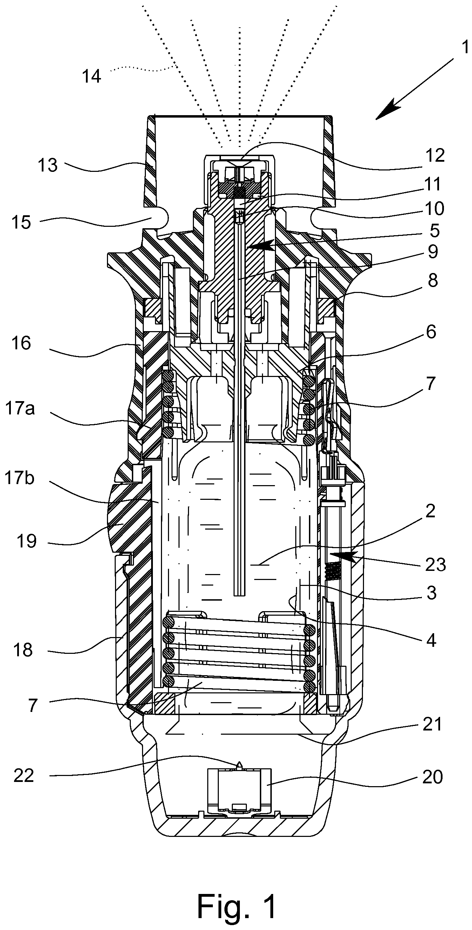

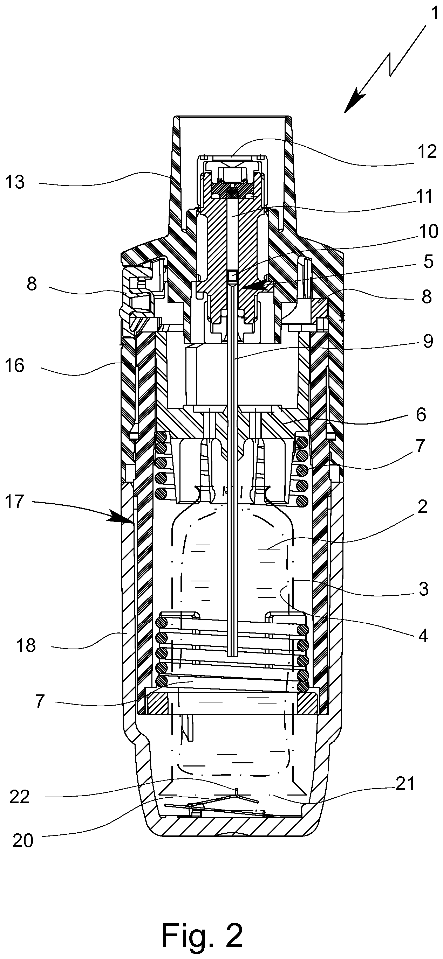

Fig. 1 und Fig. 2 zeigen einen Zerstäuber 1 zur Zerstäubung eines Fluids 2, insbesondere eines hochwirksamen Arzneimittels oder dgl., wie er beispielsweise im erfindungsgemäßen Prüfsystem oder Prüfverfahren auf seine Funktionsfähigkeit geprüft werden kann. In Fig. 1 und Fig. 2 zeigen dabei den Zerstäuber 1 in zwei Zuständen, wie sie bei Verwendung durch den Anwender auftreten: Fig. 1 und Fig. 2 zeigen den Zerstäuber 1 in einer schematischen Darstellung im ungespannten Zustand (Fig. 1) und gespannten Zustand (Fig. 2). Die Bezeichnungen "ungespannter" und "gespannter Zustand" deuten hierbei auf den Zustand des im Zerstäuber 1 enthaltenen, vorzugsweise durch eine Antriebsfeder 7 gebildeten, Energiespeichers hin. Im "gespannten Zustand" (Fig. 2) ist dieser Energiespeicher gewissermaßen geladen, der Zerstäuber 1 ist auslösebereit. Der "ungespannte Zustand" (Fig. 1) zeigt den Zerstäuber 1 im ausgelösten (nicht auslösebereiten) Zustand.

Insbesondere weist der Zerstäuber eine Auslösemöglichkeit, insbesondere eine Auslösetaste 8a auf, bei deren Betätigung die Sprühnebelerzeugung bzw. die Abgabe des Aerosols 14 automatisch bzw. selbstständig einsetzt. Vorteilsweise unterliegt die Sprühnebelerzeugung somit keinem oder nur einem vernachlässigbaren Einfluss durch die Handhabung des Zerstäubers 1 durch den Anwender / Patienten. Im vorliegenden Ausführungsbeispiel, wechselt der Zerstäuber 1 auf das Auslösen hin bzw. gestartet durch das Drücken der Auslösetaste 8a (während der Abgabe des Fluids bzw. der Sprühnebelerzeugung) von dem "gespannten" in den "ungespannten Zustand" über.

Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, mittels des Zerstäubers 1 wird vorzugsweise ein Aerosol gebildet, insbesondere wobei ein nicht dargestellter Benutzer bzw. Patient das zerstäubte Fluid 2 bzw. das Aerosol 14 inhalieren kann, vorzugsweise wobei Zuluft über mindestens eine Zuluftöffnung 15 in das Mundstück 13 saugbar ist. Üblicherweise erfolgt das Inhalieren wenigstens einmal täglich, insbesondere mehrmals täglich, vorzugsweise in vorbestimmten Zeitabständen, besonders bevorzugt in Abhängigkeit von der Erkrankung des Patienten.

Der Zerstäuber 1 weist einen vorzugsweise einsetzbaren und vorzugsweise auswechselbaren Behälter 3 mit dem Fluid 2 auf. Der Behälter 3 bildet vorzugsweise ein Reservoir für das zu zerstäubende Fluid 2.

Vorzugsweise enthält der Behälter 3 (in der Verwendung beim Anwender bzw. den in Fig. 1 und 2 gezeigten Zuständen) eine ausreichende Menge an Fluid 2 bzw. Wirkstoff, um beispielsweise bis zu 200 Dosiereinheiten zur Verfügung stellen zu können, also beispielsweise bis zu 200 Zerstäubungen oder Anwendungen zu ermöglichen. Ein typischer Behälter 3, wie in der WO 96/06011 A2 offenbart, nimmt ein Volumen von ca. 2 ml bis 10 ml auf.

Der Behälter 3 ist vorzugsweise zumindest im Wesentlichen zylindrisch bzw. kartuschenartig ausgebildet und von unten, nach Öffnen des Zerstäubers 1, in diesen einsetzbar und ggf. wechselbar.

Vorzugsweise weist der Behälter 3 an seinem Boden eine ebene Fläche auf oder hat der Behälter 3 einen ebenen Behälterboden. Optional weist der Behälter 3 eine metallische und/oder reflektierende Außenhülle und/oder einen metallischen und/oder reflektierenden Behälterboden oder eine metallische und/oder reflektierende Beschichtung (von außen) am Behälterboden auf.

Vorzugsweise ist das Fluid 2 in einem von einem kollabierbaren Beutel gebildeten Fluidraum 4 im Behälter 3 aufgenommen.

Der Zerstäuber 1 weist vorzugsweise ferner einen Druckerzeuger 5 zur Förderung und Zerstäubung des Fluids 2, insbesondere jeweils in einer vorbestimmten, ggf. einstellbaren Dosiermenge, auf.

Der Druckerzeuger 5 weist vorzugsweise eine Halterung 6 für den Behälter 3, eine zugeordnete, nur teilweise dargestellte Antriebsfeder 7 mit einem zur Entsperrung - direkt oder vorzugsweise über eine Auslösetaste 8a - manuell betätigbaren Sperrelement 8, ein Förderrohr 9 mit einem Rückschlagventil 10, eine Druckkammer 11 und eine Austragsdüse 12 im Bereich eines Mundstücks 13 auf. Vorzugsweise wird zum Auslösen des Zerstäubers das Sperrelement 8 quer zur Hauptachse des Zerstäubers 1 / quer zur Bewegungsrichtung der bewegten Teile des Druckerzeugers 5 verschoben bzw. die Auslösetaste 8a quer zur Hauptachse / Bewegungsachse eingedrückt. Vorteilsweise tragen so die für das Auslösen aufzuwendenden Kräfte nicht zu den beim Auslösen freigesetzten Kräfte bei.

Der Behälter 3 wird vorzugsweise über die Halterung 6, insbesondere rastend, so in dem Zerstäuber 1 fixiert, dass das Förderrohr 9 in den Behälter 3 eintaucht. Die Halterung 6 kann dabei derart ausgebildet sein, dass der Behälter 3 gelöst und ausgetauscht werden kann.

Beim axialen Spannen der Antriebsfeder 7 wird die Halterung 6 mit dem Behälter 3 und dem Förderrohr 9 bei den Darstellungen nach unten bewegt und das Fluid 2 aus dem Behälter 3 über das Rückschlagventil 10 in die Druckkammer 11 des Druckerzeugers 5 gesaugt.

Beim anschließenden Entspannen nach Betätigung des Sperrelements 8 wird das Fluid 2 in der Druckkammer 11 unter Druck gesetzt, indem das Förderrohr 9 mit seinem nun geschlossenen Rückschlagventil 10 durch Entspannen der Antriebsfeder 7 wieder nach oben bewegt wird und nun als Druckstempel dient. Dieser Druck treibt das Fluid 2 durch die Austragsdüse 12 aus, wobei es in ein Aerosol 14 zerstäubt wird, wie in Fig. 1 angedeutet.

Vorzugsweise ist das Förderrohr 9, insbesondere über die Halterung 6, in Gebrauchslage gegenüber dem Behälter 3 festgelegt. Es ist also insbesondere vorgesehen, dass eine (axiale) Bewegung des Förderrohrs 9 einer (axialer) Bewegung des Behälters 3 entspricht.

Wenn das Förderrohr 9 bzw. das Rückschlagventil 10 als Druckstempel fungiert, korrespondiert eine Bewegung des Förderrohrs 9 bzw. des Behälters 3 zu einem in der Drucckammer 11 verdrängten Volumen bzw. einer ausgetragenen oder austragbaren Fluidmenge.

Insbesondere unter Berücksichtigung der hydraulischen Eigenschaften des Zerstäubers 1 bzw. der Eigenschaften der Austragsdüse 12 (z.B. Düsengeometrien, hydraulische Widerstände, die durch eingebaute Filter und/oder die Austragsdüse 12 gebildet werden, etc.) können aus der Bewegung des Förderrohrs 9 bzw. des Rückschlagventils 10 und/oder eines sonstigen Druckstempels und der hierzu korrespondierenden Bewegung des Behälters 3 Rückschlüsse auf die Funktionsfähigkeit des Zerstäubers 1 gezogen werden. Bei dem hier im Ausführungsbeispiel betrachteten Zerstäuber1 wird zur Aerosolerzeugung das in der Druckkammer 11 und/oder vor der Austragsdüse 12 befindliche Fluid 2 unter Druck durch die Austragsdüse 12 und somit gegen die in diesem Fluidweg befindlichen hydraulischen Widerstände ausgetrieben. Der Fluiddruck beeinflusst dabei das entstehende Sprühbild bzw. die Eigenschaften des Aerosols 14. Auf diese Weise können anhand von Bewegungsmessung des Kolbens der Pumpe eines Zerstäubers 1 bzw. hier des Förderrohrs 9 bzw. eines daran angebrachten (vom Förderrohr 9 mitbewegten) Behälters 3 verschiedene Funktionsfehler erkannt werden, für deren Erkennung auf Seiten der Aerosolbildung sonst unter Umständen mehrere verschiedene Messmethoden notwendig wären. Beispielsweise können so durch die Bewegungsmessung sowohl Leckagen, für deren direkte Bestimmung am Aerosol andernfalls beispielsweise eine Gewichtsmessung des ausgebrachten Aerosols durchgeführt werden müsste, als auch Verlegungen oder Verstopfungen gefunden werden, die sich beispielsweise auf die Sprühzeit des Zerstäubers 1 und die Qualität des Sprühnebels auswirken (durch Verlegungen wird der hydraulische Widerstand im Zerstäuber größer, das Fluid wird langsamer aus der Druckkammer ausgetrieben). Bei Leckagen wird das Fluid nicht nur durch die Düse sondern durch zusätzliche Leckagestellen ausgetrieben, wodurch sich insgesamt der hydraulische Widerstand der Fluidwege im Zerstäuber verringert (das Fluid wird also schneller aus der Druckkammer ausgetrieben, d.h. man beobachtet eine schnellere Kolbenbewegung bzw. kürzere Sprühzeit).

Bevorzugt wird die Sprühzeit, d.h. die Zeit, in der Aerosol an der Austragsdüse 12 erzeugt wird, für einen Zerstäuber 1 mit Kolbenpumpmechanismus über der Zeit bestimmt, in der sich der Kolben (Förderrohr 9) bzw. der angeschlossene Behälter 3 auf die Auslösung des Zerstäubers 1 hin bzw. bei der Sprühnebelerzeugung bewegt. Hierbei wird vorzugsweise über eine optische Messmethode die Zeit gemessen, welche vorzugsweise ein mit dem Kolben mitbewegter Behälter 3 braucht, um sich auf Auslösung des Zerstäubers 1 hin von seiner Position im gespannten Gerätezustand zu seiner Position im relaxierten (ungespannten) Zustand zu bewegen. Die Zeit für diese Behälterbewegung korreliert oder entspricht im Wesentlichen der Sprühzeit des Zerstäubers 1, da während dieser Bewegung Flüssigkeit aus dem Zerstäuber 1 durch die Austragsdüse 12 nach außen gepresst wird.

Der Zerstäuber 1 weist vorzugsweise ein Gehäuseteil 16 und ein demgegenüber drehbares Innenteil 17 (Fig. 2) mit einem oberen Teil 17a und einem unteren Teil 17b (Fig. 1) auf, insbesondere wobei an dem Innenteil 17 ein insbesondere manuell betätigbares Gehäuseunterteil bzw. eine Kappe 18 vorzugsweise mittels eines Halteelementes 19 lösbar befestigt, insbesondere aufgesteckt, ist.

Zum Einsetzen und/oder Auswechseln des Behälters 3 ist das Gehäuseunterteil 18 vorzugsweise vom Zerstäuber 1 lösbar.

Das Gehäuseunterteil 18 kann gegen das Gehäuseteil 16 gedreht werden, vorzugsweise wobei es den in der Darstellung unteren Teil 17b des Innenteils 17 mitnimmt. Dadurch wird die Antriebsfeder 7 über ein nicht dargestelltes, auf die Halterung 6 wirkendes Getriebe in axialer Richtung gespannt. Mit dem Spannen wird der Behälter 3 vorzugsweise axial nach unten bewegt, bis der Behälter 3 eine in Fig. 2 angedeutete Endlage annimmt. In diesem Zustand ist die Antriebsfeder 7 gespannt.

Vorzugsweise kommt bei Verwendung des Zerstäubers durch den Anwender bzw. Patienten beim erstmaligen Spannen eine axial wirkende, im Gehäuseunterteil 18 angeordnete Feder 20 am Behälterboden 21 zur Anlage und sticht mit einem Anstechelement 22 den Behälter 3 bzw. eine bodenseitige Versiegelung bei der erstmaligen Anlage zur Belüftung an. Während des Zerstäubungsvorgangs wird der Behälter 3 vorzugsweise von der Antriebsfeder 7 wieder in seine Ausgangslage zurückbewegt.

Der Behälter 3 führt vorzugsweise eine Hubbewegung während des Spannvorgangs bzw. zur Fluidentnahme und/oder während der Zerstäubung bzw. Abgabe des Fluids 2 aus. Besonders bevorzugt bewegt sich der Behälter 3 während der Zerstäubung bzw. Abgabe des Fluids 2 um den Hub bzw. Hubweg Δs_{Hub}, mit der - vorzugsweise zumindest teilweise linearen - Geschwindigkeit v_{Hub} und innerhalb der Zeit Δt_{Hub}, vorzugsweise wobei die Zeit Δt_{Hub} zumindest im Wesentlichen derjenigen Zeit entspricht, innerhalb derer das Fluid 2 abgegeben bzw. zerstäubt wird.

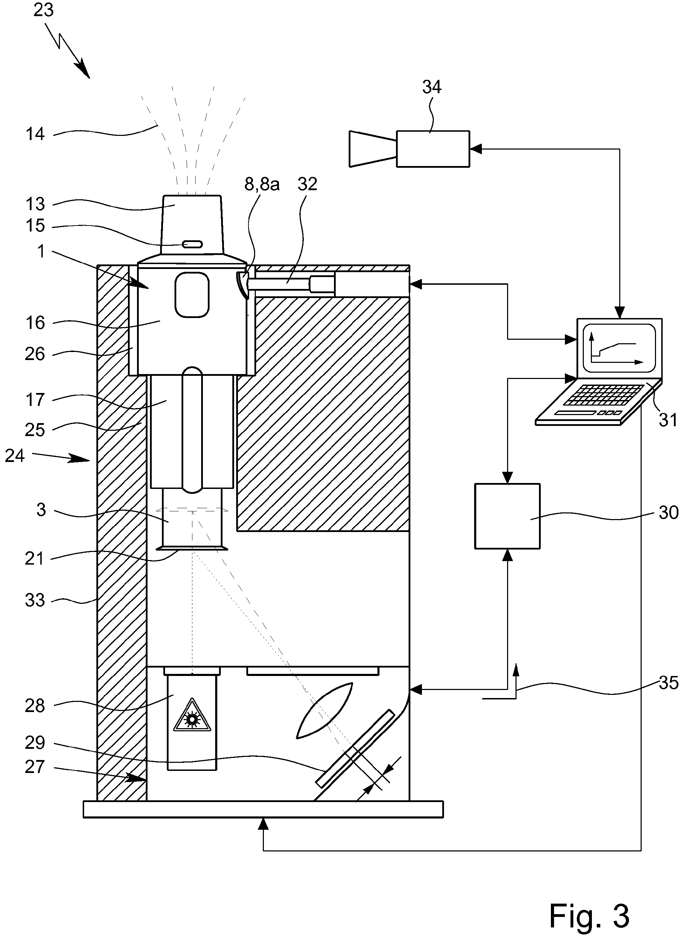

Fig. 3 zeigt schematisch ein vorschlagsgemäßes System 23 zum Prüfen der Funktionsfähigkeit des Zerstäubers 1.

Das vorschlagsgemäße System bzw. Prüfsystem 23 weist vorzugsweise den Zerstäuber 1 bzw. zumindest Bauteile oder Baugruppen des Zerstäubers 1 und eine vorschlagsgemäße Prüfvorrichtung 24 auf.

Der Zerstäuber 1 weist einen vorzugsweise einsetzbaren und vorzugsweise auswechselbaren Behälter 3 mit dem Fluid 2 auf. Der Behälter 3 bildet vorzugsweise ein Reservoir für das beim Prüfen zu zerstäubende Fluid 2.

Der Behälter 3 ist vorzugsweise zumindest im Wesentlichen zylindrisch bzw. kartuschenartig ausgebildet und in den Zerstäuber 1 einsetzbar und wechselbar bzw. entfernbar. Im Darstellungsbeispiel ist ein Teil des Behälters 3 im Prüfsystem 23 vorzugsweise direkt für Messungen zugänglich.

Der Behälter 3 ist vorzugsweise zumindest im Wesentlichen starr ausgebildet. Besonders bevorzugt ist der Behälter 3 aus Kunststoff, insbesondere thermoplastischen Kunststoff, ganz besonders bevorzugt Polyetheretherketon, hergestellt. Vorzugsweise weist der Behälter 3 an seinem Boden eine ebene Fläche auf oder hat der Behälter 3 einen ebenen Behälterboden.

Optional weist der Behälter 3 eine metallische und/oder reflektierende Außenhülle und/oder einen metallischen und/oder reflektierenden Behälterboden 21 oder eine metallische und/oder reflektierende Beschichtung (von außen) am Behälterboden 21 auf.

Die Prüfvorrichtung 24 ist vorzugsweise dazu ausgebildet, den Zerstäuber 1 zu prüfen, insbesondere hinsichtlich seiner Funktionsfähigkeit und/oder auf etwaige Fehler.

Insbesondere ist mittels des Systems 23 bzw. der Prüfvorrichtung 24 prüfbar bzw. feststellbar, ob und/oder inwieweit der Zerstäuber 1 funktionsfähig ist und/oder vordefinierte Anforderungen erfüllt bzw. vordefinierte Funktionen aufweist und/oder die Funktion des Zerstäubers 1 den für ihn bzw. individuell definierten Spezifikationen entspricht.

Besonders bevorzugt ist das System 23 bzw. die Prüfvorrichtung 24 dazu ausgebildet, fehlerhafte bzw. in ihrer Funktion beeinträchtigte Zerstäuber 1 zu identifizieren, vorzugsweise im Rahmen der Fertigung bzw. der Montage der Zerstäuber 1. Ganz besonders bevorzugt sind mittels des Systems 23 bzw. der Prüfvorrichtung 24 fehlerhafte bzw. in ihrer Funktion beeinträchtigte Zerstäuber 1 identifizierbar und/oder (anschließend) aussortierbar.

Vorzugsweise ist das System 23 bzw. die Prüfvorrichtung 24 in die Fertigung bzw. den Fertigungsprozess, insbesondere den Montageprozess, des Zerstäubers 1 integriert oder integrierbar.

Das System 23 bzw. die Prüfvorrichtung 24 weist vorzugsweise eine Halteeinrichtung 25 auf. Die Halteeinrichtung 25 ist vorzugsweise dazu ausgebildet, den Zerstäuber 1 bzw. das Gehäuseteil 16 und/oder den Innenteil 17 zu halten, einzuspannen und/oder aufzunehmen, insbesondere derart, dass der Zerstäuber 1 bzw. zumindest das Gehäuseteil 16 und/oder das Innenteil 17 bei der Abgabe des Fluids 2 relativ zur Halteeinrichtung 25 unbeweglich ist.

Vorzugsweise weist die Halteeinrichtung 25 eine Aufnahme 26 auf, in die der Zerstäuber 1 zumindest teilweise aufgenommen oder aufnehmbar bzw. eingesetzt oder einsetzbar ist.

Das System 23 bzw. die Prüfvorrichtung 24 weist vorzugsweise eine Messeinrichtung 27 auf, vorzugsweise wobei die Messeinrichtung 27 dazu ausgebildet ist, die Bewegung des Kolbens (Förderrohrs 9) oder des Behälters 3 bei der Abgabe des Fluids 2 zu messen.

Die Messeinrichtung 27 ist vorzugsweise als optische, mechanische und/oder elektrische Messeinrichtung ausgebildet.

Insbesondere ist die Messeinrichtung 27 dazu ausgebildet, die Bewegung des Behälters 3 bzw. des Behälterbodens 21, insbesondere die Geschwindigkeit v_{Hub}, den Hub Δs_{Hub} und/oder die Zeit Δt_{Hub} des Hubs, berührungslos, optisch, mechanisch und/oder elektrisch zu messen.

Besonders bevorzugt ist die Messeinrichtung 27 dazu ausgebildet, die Bewegung des Behälters 3 bzw. des Behälterbodens 21, insbesondere die Geschwindigkeit v_{Hub}, den Hub Δs-_{Hub} und/oder die Zeit Δt_{Hub} des Hubs mittels Triangulation zu messen. Insbesondere beinhaltet die Messeinrichtung 27 ein Laser-Triangulations-Sensor-System oder wird von einem solchen gebildet.

Vorzugsweise weist die Messeinrichtung 27 einen Emitter 28, insbesondere einen Laser oder eine andere Quelle, die Strahlung bzw. elektromagnetische Wellen abgibt, einen Sensor 29 und optional einen Analog-Digital-Wandler auf, vorzugsweise wobei mittels des Analog-Digital-Wandlers von dem Sensor 29 erfasste Strahlung, Bilder oder andere detektierte Signale in ein oder mehrere elektrische Signale umgewandelt werden.

Der Sensor 29 kann insbesondere dazu ausgebildet sein, eine Lage oder Position und/oder einen Winkel und/oder eine Intensität der einfallenden Strahlung bzw. elektromagnetischen Wellen zu detektieren bzw. in Daten bzw. Datensignale, wie Messwerte bzw. Messsignale, insbesondere der Messeinrichtung 27, zu wandeln.

Insbesondere handelt es sich bei dem Sensor 29 um einen CCD-Sensor, CMOS-Sensor oder dergleichen.

Der Sensor 29 weist vorzugsweise mehrere voneinander unterscheidbare, bevorzugt nebeneinander angeordnete Sensorabschnitte, sogenannte Pixel, auf. Hierdurch kann die Position bzw. Lage einfallender elektromagnetischer Wellen bzw. einfallenden Lichts bestimmt werden.

Der Sensor 29 korrespondiert vorzugsweise zum Emitter 28 derart, dass der Sensor 29 dazu geeignet ist, von dem Emitter 28 ausgesendete elektromagnetische Wellen bzw. Strahlung zu empfangen, zu detektieren und/oder zu wandeln.

Der Sensor 29 kann eine optische Einrichtung, wie eine Linse, aufweisen, die vorzugsweise eine Fokussierung von elektromagnetischen Wellen bewirkt, um die Lage und/oder den Einfallswinkel und/oder die Intensität einfallender elektromagnetischer Wellen zu verändern bzw. zu präzisieren (insbesondere zur Verbesserung der Auswertung und/oder Genauigkeit am Sensor 29).

Der Emitter 28 und der Sensor 29 sind vorzugsweise derart zueinander angeordnet und dazu ausgebildet, dass eine Bewegung des Behälters 3 bzw. des Behälterbodens 21 zu einer Änderung der Einfallsposition, des Einfallswinkels und/oder der Einfallsintensität von durch den Emitter 28 ausgesendeten elektromagnetischen Wellen bzw. Strahlung führt. Dies hat sich als besonders vorteilhaft für eine genaue Bestimmung der Lage bzw. Position, Bewegung und/oder Geschwindigkeit des Behälters 3 bzw. des Behälterbodens 21 erwiesen.

Der Emitter 28 ist insbesondere dazu ausgebildet, elektromagnetische Wellen bzw. Strahlung, insbesondere Laserstrahlung, zu emittieren, vorzugsweise in Richtung des Zerstäubers 1, insbesondere des Behälters 3, besonders bevorzugt des Behälterbodens 21. Bevorzugt beinhaltet der Emitter eine Laserdiode oder wird von einer solchen gebildet.

Die Messeinrichtung 27 bzw. der Emitter 28 ist vorzugsweise unterhalb des Zerstäubers 1, insbesondere des Behälters 3 bzw. des Behälterbodens 21, angeordnet und/oder derart ausgerichtet, dass der Zerstäuber 1, insbesondere der Behälter 3 bzw. der Behälterboden 21, mittels des Emitters 28 bestrahlbar ist.

Insbesondere ist mittels des Behälters 3 bzw. Behälterbodens 21 die vom Emitter 28 emittierte Strahlung reflektierbar, vorzugsweise zumindest teilweise in Richtung des Sensor 29. Optional weist der Behälter 3 bzw. der Behälterboden 21 eine Reflexionsschicht zur Reflektion der vom Emitter 28 emittierten Strahlung auf (nicht dargestellt).

Vorzugsweise ist der Emitter 28 derart relativ zum Zerstäuber 1 angeordnet, dass die vom Emitter 28 emittierte Strahlung nahezu orthogonal zum Behälterboden 21 und/oder nahezu parallel zur Bewegungsachse des Behälters 3 verläuft, wie in Fig. 3 angedeutet.

Insbesondere sind Emitter 28 und Sensor 27 relativ zum Behälterboden 21 derart angeordnet, dass nach dem Prinzip, dass Einfallswinkel gleich Ausfallswinkel ist, die vom Emitter 28 emittierte Strahlung vom Behälterboden 21 zum Sensor 27 hin reflektiert wird, vorzugsweise wobei Emitter 28 und Sensor 27 vorzugsweise nah beieinander angeordnet sind.

Bei der dargestellten Ausführungsform ist das Gehäuseunterteil 18 des Zerstäubers 1 zum Prüfen bzw. zur Messung mittels der Prüfvorrichtung 24 abgenommen bzw. ist der Zerstäuber 1 ohne Gehäuseunterteil 18 in der Halteeinrichtung 25 eingespannt bzw. aufgenommen. Auf diese Weise wird eine Streuung der vom Emitter 28 emittierten Strahlung reduziert. Es sind jedoch auch Lösungen möglich, bei denen der Zerstäuber 1 zusammen mit dem Gehäuseunterteil 18 messbar ist oder gemessen wird. Insbesondere sind Lösungen möglich, bei denen der Zerstäuber 1 im (vollständig) fertig montierten Zustand prüfbar ist oder geprüft wird. Beispielsweise kann das Gehäuseunterteil bzw. die Kappe 18 transparent bzw. für die emittierte und reflektierte Strahlung durchlässig ausgebildet sein. Der Sensor 29 ist vorzugsweise dazu ausgebildet, die vom Emitter 28 emittierte und/oder am Behälter 3 bzw. Behälterboden 21 reflektierte Strahlung zumindest teilweise zu detektieren.

Durch die Reflektion der Strahlung am Behälter 3 bzw. Behälterboden 21 und die Detektion der reflektierten Strahlung mittels des Sensors 29 ist es möglich, den Abstand bzw. den sich beim Zerstäubungsvorgang verändernden Abstand zwischen der Messeinrichtung 27 bzw. einer dem Zerstäuber 1 zugewandten Seite der Messeinrichtung 27 und dem Behälter 3 bzw. dem Behälterboden 21 und/oder bei einer Hubbewegung den Hub Δs_{Hub} des Behälters 3 bzw. die Abstandsänderung zu bestimmen.

Zusätzlich oder alternativ zur Triangulation können auch andere optische Messverfahren bzw. -prinzipien, wie die Interferometrie, das Schattenbildverfahren und/oder die kamerabasierte Segmentierung, zum Einsatz kommen. Insbesondere kann die Bewegung des Behälters 3 bzw. des Behälterbodens 21, besonders bevorzugt die Geschwindigkeit v_{Hub}, der Hub Δs_{Hub} und/oder die Zeit Δt_{Hub} des Hubs, - zusätzlich oder alternativ zur Triangulation - mittels anderer optischer Messverfahren, wie der Interferometrie, des Schattenbildverfahrens und/oder der kamerabasierten Segmentierung, gemessen werden.

Beispielsweise kann die Prüfvorrichtung 24 bzw. die Messeinrichtung 27 einen (weiteren) Emitter und einen (weiteren) Sensor aufweisen, vorzugweise wobei der Behälter 3 zwischen dem Emitter und dem Sensor angeordnet ist und/oder derart vom Emitter beleuchtet wird, dass ein Schatten bzw. ein Schattenbild des Behälters 3 bzw. eine Bewegung des Behälters 3 bei der Abgabe des Fluids 2 über die Änderung des Schattens bzw. des Schattenbilds mittels des Sensors detektiert wird.

Gemäß einer weiteren Ausführungsform (nicht dargestellt) weist der Behälter 3 vorzugsweise seitlich mindestens eine Markierung, insbesondere Linienmarkierung, auf, vorzugsweise wobei die Bewegung des Behälters 3 bei der Abgabe des Fluids 2 über die Markierung und eine zugeordnete (weitere) Aufnahmeeinrichtung, insbesondere eine Kamera, gemessen bzw. verfolgt wird.

Vorzugsweise ist bei einer derartigen Ausführungsform die Datenverarbeitungseinrichtung 31 dazu ausgebildet, die Position der Markierung vor, während und/oder nach der Abgabe des Fluids 2 zu ermitteln und/oder eine Segmentierung, insbesondere eine Kantendetektion, der Aufnahme des Behälters 3 bzw. der Markierung durchzuführen.

Zusätzlich oder alternativ zu optischen Messverfahren bzw. -prinzipien können auch mechanische und/oder elektrische Messverfahren bzw. -prinzipien zum Einsatz kommen. Insbesondere kann die Bewegung des Behälters 3 bzw. des Behälterbodens 21, insbesondere die Geschwindigkeit v_{Hub}, der Hub Δs_{Hub} und/oder die Zeit Δt_{Hub} des Hubs, - zusätzlich oder alternativ zu optischen Messverfahren - mittels mechanischer und/oder elektrischer Messverfahren gemessen werden.

Beispielsweise kann die Bewegung und/oder Position des Behälters 3 vor, während und/oder nach Abgabe des Fluids 2 mittels mindestens eines taktilen Sensors bzw. eines Messtasters erfasst werden.

Bei einer weiteren Ausführungsform (nicht dargestellt) kann die Bewegung des Behälters 3 bzw. eine Abstandsänderung des Behälters 3 induktiv bzw. mittels eines induktiven Sensors gemessen werden.

Vorzugsweise weist der Behälter 3 bei einer derartigen Ausführungsform ein elektrisch leitfähiges Material, wie Metall, beispielsweise am Behälterboden 21, auf und/oder besteht der Behälter 3 bei einer derartigen Ausführungsform zumindest teilweise aus einem elektrisch leitfähigen Material, wie Metall, vorzugsweise wobei eine veränderte Induktivität bei einer Bewegung des Behälters 3 bzw. Abstandsänderung des Behälters 3 zu einem zugeordneten Sensor detektierbar ist bzw. detektiert wird.

Bei einer weiteren Ausführungsform (nicht dargestellt) kann die Bewegung des Behälters 3 bzw. eine Abstandsänderung des Behälters 3 kapazitiv bzw. mittels eines kapazitiven Sensors gemessen werden, vorzugsweise wobei eine veränderte Kapazität bei einer Bewegung des Behälters 3 bzw. Abstandsänderung des Behälters 3 zu einem zugeordneten Sensor detektierbar ist bzw. detektiert wird.

Vorzugsweise werden mehrere, insbesondere unterschiedliche, Messverfahren bzw. - prinzipien miteinander kombiniert. In vorteilhafter Weise können so etwaige Messfehler erkannt und die Messgenauigkeit erhöht werden.

Bevorzugt werden Hubwege bzw. Hübe Δs_{Hub} in der Größenordnung von 0,5 mm bis 100 mm, besonders bevorzugt von 1 mm bis 20 mm, insbesondere von 4 mm bis 12 mm, gemessen.

Vorzugsweise beträgt der Abstand zwischen der Messeinrichtung 27, insbesondere einer dem Zerstäuber 1 zugewandten Seite der Messeinrichtung 27, bzw. dem Emitter 28 und dem Zerstäuber 1 bzw. dem Behälter 3 bzw. dem Behälterboden 21 im gespannten Zustand des Zerstäubers 1 mehr als 5 mm oder 10 mm, besonders bevorzugt mehr als 20 mm oder 30 mm, insbesondere mehr als 40 mm, und/oder weniger als 200 mm oder 150 mm, besonders bevorzugt weniger als 100 mm, insbesondere weniger als 80 mm.

Das System 23 bzw. die Prüfvorrichtung 24 weist vorzugsweise eine Steuereinrichtung 30 auf, vorzugsweise wobei die Steuereinrichtung 30 dazu ausgebildet ist, die Messeinrichtung 27, insbesondere den Emitter 28 und/oder Sensor 29, zu steuern und/oder zu regeln.

Vorzugsweise weist das System 23 bzw. die Prüfvorrichtung 24 eine Datenverarbeitungseinrichtung 31, wie einen Computer, auf, vorzugsweise wobei die Datenverarbeitungseinrichtung 31 dazu ausgebildet ist, Daten bzw. Datensignale, wie Messwerte bzw. Messsignale, insbesondere der Messeinrichtung 27, zu verarbeiten, zu speichern, auszuwerten und/oder mit Sollwerten, insbesondere einer Sollgeschwindigkeit, einem Sollhub und/oder einer Sollzeit, und/oder Grenzwerten, insbesondere einer maximalen und/oder minimalen Geschwindigkeit, einem maximalen und/oder minimalen Hub und/oder einer maximalen und/oder minimalen Zeit, zu vergleichen.

Die Datenverarbeitungseinrichtung 31 ist dazu ausgebildet, einen fehlerhaften Zerstäuber 1 zu identifizieren bzw. einen Zerstäuber 1 (automatisch) als fehlerhaft zu identifizieren bzw. zu markieren, wenn die von der Messeinrichtung 27 ermittelten Messwerte nicht den Sollwerten, insbesondere einer Sollgeschwindigkeit, einem Sollhub und/oder einer Sollzeit, entsprechen und/oder außerhalb eines Bereichs liegen, der durch die Grenzwerte, wie eine maximale und/oder minimale Geschwindigkeit, einen maximalen und/oder minimalen Hub und/oder eine maximale und/oder minimale Zeit, bestimmt ist.

Besonders bevorzugt ist die Messeinrichtung 27 elektrisch mit der Steuereinrichtung 30 und/oder der Datenverarbeitungseinrichtung 31 verbunden oder verbindbar.

Vorzugsweise weist das System 23 bzw. die Prüfvorrichtung 24 eine Betätigungs- bzw. Auslöseeinrichtung 32, wie einen Aktuator, auf, vorzugsweise wobei die Betätigungseinrichtung 32 dazu ausgebildet ist, den Zerstäuber 1 bzw. das Sperrelement 8 oder die Auslösetaste 8a des Zerstäubers 1 zu betätigen und/oder die Abgabe des Fluids 2 auszulösen.

Vorzugsweise ist die Betätigungseinrichtung 32 als elektrischer Antrieb ausgebildet. Hier sind jedoch auch andere Lösungen möglich. Optional weist die Auslöseeinrichtung 32 eine Kraftmesseinrichtung (nicht dargestellt) auf, insbesondere wobei die Kraftmesseinrichtung dazu ausgebildet ist, diejenige Kraft zu messen, die notwendig ist, um das Sperrelement 8 für das Auslösen des Zerstäubers 1 zu bewegen bzw. um die Auslösetaste 8a zum Auslösen einzudrücken. Insbesondere wird dabei die Auslösekraft in Abhängigkeit von der Zeitdauer des Auslöse- oder Betätigungsvorgangs gemessen.

Vorzugsweise ist die Betätigungseinrichtung 32 elektrisch mit der Steuereinrichtung 30 und/oder Datenverarbeitungseinrichtung 31 verbunden und/oder mittels der Steuereinrichtung 30 und/oder Datenverarbeitungseinrichtung 31 steuerbar, insbesondere auslösbar.

Optional weist das System 23 bzw. die Prüfvorrichtung 24 eine (nicht dargestellte) Waage auf, vorzugsweise wobei die Waage dazu ausgebildet ist, das Gewicht des abgegeben Fluids 2 zu bestimmen. Besonders bevorzugt ist die Waage in die Halterung des Zerstäubers 1 in der Prüfvorrichtung 24 integriert (nicht dargestellt).Vorzugsweise ist die Waage elektrisch mit der Steuereinrichtung 30 und/oder der Datenverarbeitungseinrichtung 31 verbunden.

Vorzugsweise weisen die Halteeinrichtung 25, die Messeinrichtung 27, die Steuereinrichtung 30, die Betätigungseinrichtung 32 und/oder die Waage ein gemeinsames Gehäuse 33 auf und/oder sind die Halteeinrichtung 25, die Messeinrichtung 27, die Steuereinrichtung 30, die Betätigungseinrichtung 32 und/oder die Waage in ein gemeinsames Gehäuse 33 integriert. Auf diese Weise wird eine besonders kompakte Bauweise ermöglicht oder unterstützt.

Optional weist das System 23 bzw. die Prüfvorrichtung 24 eine Spanneinrichtung (nicht dargestellt) auf, vorzugsweise wobei die Spanneinrichtung dazu ausgebildet ist, den Zerstäuber 1 zu spannen bzw. das Gehäuseteil 16 relativ zum Innenteil 17 bzw. Gehäuseunterteil 18 zu verdrehen. Ganz besonders bevorzugt ist der Zerstäuber 1 mittels der Prüfvorrichtung 24 spannbar und betätigbar. Vorzugsweise weist die Spanneinrichtung eine Messeinrichtung auf, wobei die Messeinrichtung dazu ausgebildet ist, die Kraft zu messen, die notwendig ist, um den Zerstäuber 1 zu spannen, und/oder das Drehmoment zu messen, das überwunden werden muss, um das Gehäuseteil 16 relativ zum Innenteil 17 bzw. Gehäuseunterteil 18 zum Laden des Energiespeichers zu verdrehen. Vorzugsweise ist die Prüfvorrichtung 24 dazu ausgebildet, dass mehrere Prüfzyklen bestehend aus Spannen des Zerstäubers 1 und Auslösen (Betätigung des Sperrelements 8 bzw. der Auslösetaste 8a des Zerstäubers 1) und insgesamt mindestens einer Funktionsfähigkeitsmessung während der Zerstäubung automatisiert an der Prüfvorrichtung 24 durchgeführt werden können.

Optional weist das System 23 bzw. die Prüfvorrichtung 24 eine Absaugung (nicht dargestellt) auf oder ist das System 23 bzw. die Prüfvorrichtung 24 mit einer Absaugung verbunden, wobei die Absaugung oder ein Anschluss zu der Absaugung dazu ausgebildet ist, - vorzugsweise während oder nach der Aerosolerzeugung durch den Zerstäuber 1 - das vom Zerstäuber 1 abgegebene Aerosol insbesondere aus der Prüfvorrichtung 24 heraus abzusaugen und/oder abzuleiten.

Optional weist das System 23 bzw. die Prüfvorrichtung 24 eine Aufnahmeeinrichtung 34 auf, vorzugsweise wobei die Aufnahmeeinrichtung 34 dazu ausgebildet ist, den Zerstäuber 1, insbesondere das Aerosol 14, bei der Abgabe optisch oder bildlich aufzunehmen bzw. die Abgabe des Aerosols 14 optisch oder in Form von Abbildungen zu dokumentieren.

Die Aufnahmeeinrichtung 34 ist vorzugsweise elektrisch mit der Steuereinrichtung 30 und/oder der Datenverarbeitungseinrichtung 31 verbunden oder verbindbar.

Eine "Aufnahmeeinrichtung" im Sinne der vorliegenden Erfindung ist eine insbesondere optische, foto-, film- und/oder videotechnische Einrichtung, die vorzugsweise dazu ausgebildet ist, eine insbesondere digitale, statische, fotografische und/oder optische Aufnahme, wie ein Video, eine Videosequenz, ein Bild bzw. Foto, eine Bildabfolge, ein Standbild o. dgl., eines Gegenstands, insbesondere des abgegebenen Fluids 2 bzw. des Aerosols 14, aufzunehmen, aufzuzeichnen, zu erzeugen, zu bearbeiten, zu speichern und/oder zu übermitteln bzw. zu versenden. Vorzugsweise ist eine Aufnahmeeinrichtung im Sinne der vorliegenden Erfindung eine Kamera, insbesondere eine Film- und/oder Videokamera, Webcam, Screencam, Digicam, Camcorder o. dgl., und/oder ein Fotoapparat und/oder weist eine Aufnahmeeinrichtung eine solche bzw. einen solchen und/oder ein Kameramodul auf.

Im Folgenden soll das erfindungsgemäße Verfahren zum Prüfen der Funktionsfähigkeit des Zerstäubers 1 näher erläutert werden.

Das vorschlagsgemäße Prüfverfahren wird vorzugsweise mit dem vorschlagsgemäßen System 23 bzw. der Prüfvorrichtung 24 durchgeführt. Insbesondere ist das System 23 bzw. die Prüfvorrichtung 24 dazu ausgebildet, das vorschlagsgemäße Verfahren auszuführen.

Bei dem Prüfverfahren wird der Zerstäuber 1 hinsichtlich seiner Funktionsfähigkeit und/oder auf etwaige Fehler geprüft. Insbesondere wird festgestellt, ob und/oder inwieweit der Zerstäuber 1 funktionsfähig ist und/oder vordefinierte Anforderungen erfüllt bzw. vordefinierte Funktionen aufweist.

Besonders bevorzugt werden fehlerhafte bzw. in ihrer Funktion beeinträchtigte Zerstäuber 1 identifiziert und/oder (anschließend) aussortiert. Vorzugsweise wird das Verfahren während oder im Anschluss an den Fertigungsprozess, insbesondere den Montageprozess, des Zerstäubers 1 durchgeführt.

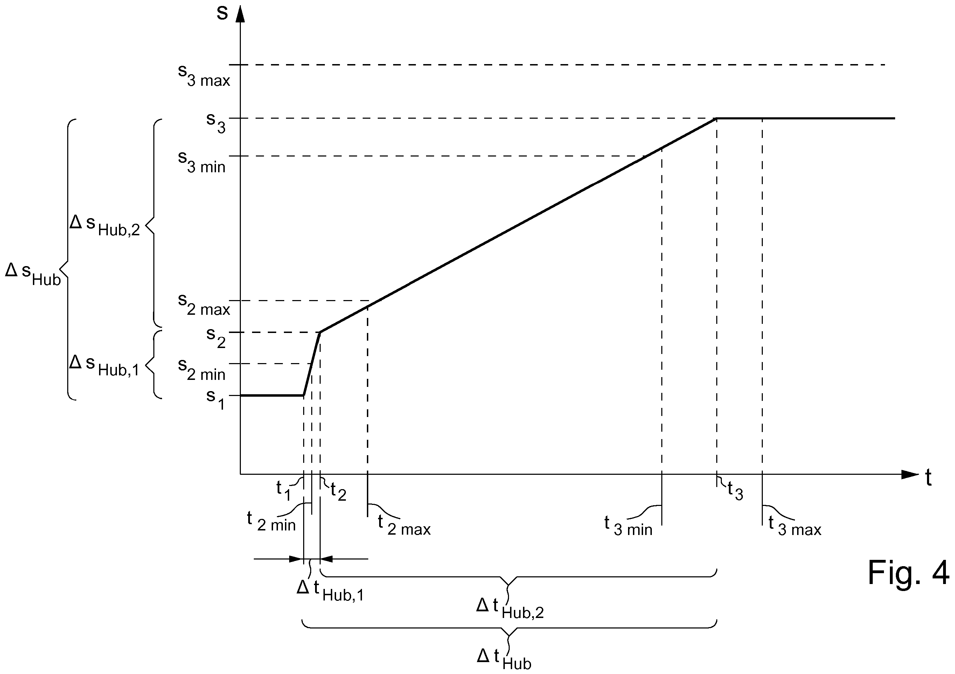

Fig. 4 zeigt einen schematischen bzw. idealisierten Verlauf des Hubs bzw. der vom Behälter 3 zurückgelegten Strecke s in Abhängigkeit von der Zeit t, vorzugsweise wobei der Zerstäuber 1 im Zeitpunkt t₁ betätigt wird bzw. die Hubbewegung des Behälters 3 beginnt und im Zeitpunkt t₃ die Hubbewegung des Behälters 3 bzw. die Abgabe des Fluids 2 endet.

Wie Fig. 4 veranschaulicht, lässt sich die Hubbewegung, insbesondere der Hub Δs_{Hub} und die Hubzeit Δt_{Hub}, des Behälters 3 grundsätzlich in zwei Bereiche unterteilen.

In einem ersten Bereich t₁ bis t₂ bzw. s₁ bis s₂ bewegt sich der Behälter 3 vorzugsweise mit einer höheren Geschwindigkeit v_{Hub} als in einem zweiten Bereich t₂ bis t₃ bzw. s₂ bis s₃. Der Behälter 3 bewegt sich in dem ersten Bereich vorzugsweise um den Leerhub Δs_{Hub,1} in der Zeit Δt_{Hub,1}. In dem ersten Bereich wird vorzugsweise kein oder nur wenig Fluid 2 abgegeben bzw. zerstäubt. Dies ist insbesondere auf die Kompression von möglichen Luftblasen im Zerstäuber 1, der elastischen Verformung des Zerstäubers 1 bzw. von Bauteilen des Zerstäubers 1 etc. zurückzuführen.

In dem auf den ersten Bereich folgenden zweiten Bereich bewegt sich der Behälter 3 vorzugsweise um den Nutzhub Δs_{Hub,2} in der Zeit Δt_{Hub,2}, vorzugsweise wobei das Fluid 2 abgegeben bzw. zerstäubt wird.

Vorzugsweise ist der Leerhub Δs_{Hub,1} und/oder die Zeit Δt_{Hub,1} wesentlich kürzer als die Zeit Δt_{Hub,2}. Besonders bevorzugt ist die Zeit Δt_{Hub,1} im Vergleich zur Zeit Δt_{Hub,2} vernachlässigbar kurz.

Zu den möglichen und/oder mittels des Prüfverfahrens messbaren, identifizierbaren, quantifizierbaren, abschätzbaren bzw. indizierbaren Fehlern des Zerstäubers 1 zählen insbesondere ein zu kurzer Hub Δs_{Hub} bzw. Nutzhub Δs_{Hub,2} des Behälters 3, eine zu hohe und/oder zu niedrige Geschwindigkeit v_{Hub} des Behälters 3, ein zu langer Leerhub Δs_{Hub,1} des Behälters 3, eine zu kurze und/oder zu lange Sprühzeit Δt_{Hub} des Zerstäubers 1. Bevorzugt werden dabei - vorzugsweise indirekt - als Funktionsbeeinträchtigungen des Zerstäubers 1 ein zu hohes Leckagevolumen bzw. eine zu hohe Leckagemasse des Fluids 2 bei der Abgabe des Fluids 2, zu hohe Druckverluste im Zerstäuber 1, insbesondere im Förderrohr 9 und/oder der Austragsdüse 12, ein zu niedriges Abgabevolumen bzw. eine zu niedrige Abgabemasse des abgegebenen Fluids 2 bzw. Aerosols 14, eine zu niedrige Strömungsgeschwindigkeit des Fluids 2 in der Austragsdüse 12 und eine zu niedrige oder zu hohe Austrittsgeschwindigkeit des Aerosols 14 erkannt. Beispiele für mögliche Ursachen für Leckagen, die so anhand einer verkürzten Sprühzeit aufgespürt werden können, sind fehlerhafte Dichtungen oder Montagefehler bei der Zusammenfügung der Komponenten, die den Fluidweg im Zerstäuber 1 bestimmen. Funktionsbeeinträchtigende Ablagerungen oder Verlegungen im Fluidweg (insbesondere in Filter oder Düse) würden dagegen anhand von verlängerter Sprühzeit aufgespürt werden.

Vorzugsweise werden der Hub Δs_{Hub} des Behälters 3, insbesondere der Leerhub Δs_{Hub,1} und der Nutzhub Δs_{Hub,2} des Behälters 3, die Geschwindigkeit v_{Hub} des Behälters 3 und die Sprüh- bzw. Hubzeit Δt_{Hub} des Zerstäubers 1 direkt bzw. unmittelbar mittels des Prüfverfahrens gemessen bzw. mit entsprechenden Messwerten erfasst. Vorzugsweise werden die Hubzeit Δt_{Hub} und die Steigung einer Ausgleichsgeraden zum zeitabhängig gemessenen Hubwegverlauf im zweiten Bereich, d.h. im Zeitbereich t₂ bis t₃, als Prüfparameter verwendet.

Vorzugsweise werden anschließend die gemessenen Werte (vorzugsweise nicht nur die an der Messeinrichtung 27 sondern auch an anderen Mess- oder Aufnahmeeinrichtungen der Prüfvorrichtung 24 ermittelten Werte oder Daten) mit - vorzugsweise empirisch, numerisch, theoretisch und/oder praktisch ermittelten - Sollwerten und/oder -bereichen bzw. Grenzwerten verglichen.

Der Zerstäuber 1 wird (automatisch) aussortiert bzw. als zumindest im Wesentlichen fehlerhaft oder funktionsunfähig identifiziert bzw. klassifiziert, wenn die gemessenen Werte nicht den Sollwerten entsprechen und/oder nicht innerhalb der Sollbereiche liegen.

Wenn die Prüfvorrichtung 24 neben der Messeinrichtung 27 zur Messung der Bewegung des Behälters 3 noch weitere (nicht dargestellte) Messeinrichtungen, z.B. eine Waage und/oder eine Messeinrichtung zur Bestimmung der Kraft zur Betätigung der Auslösetaste 8a und/oder eine Messeinrichtung zur Bestimmung der zum Spannen des Zerstäubers 1 benötigten Kraft, aufweist, so findet vorzugsweise ebenfalls ein Vergleich der dort gemessenen Werte (z.B. Gewichts- oder Kraftwerte) mit zuvor ermittelten und/oder spezifizierten Sollwerten und/oder Sollbereichen bzw. Grenzwerten und eine Aussortierung von anhand dieses Vergleichs als fehlerhaft identifizierten Zerstäubern 1 statt. Vorzugsweise werden auch optische, fotografische oder bildliche Daten, die mit der (optionalen) Aufnahmeeinrichtung 34 erfasst werden, ausgewertet und mit entsprechenden als grenzwertig definierten Daten bzw. Bildsätzen verglichen und die anhand eines solchen Vergleichs als fehlerhaft identifizierten Zerstäuber 1 aussortiert.

Vorzugsweise wird der Zerstäuber 1 (automatisch) als funktionsfähig oder zumindest im Wesentlichen fehlerfrei identifiziert bzw. klassifiziert, wenn die gemessenen Werte den Sollwerten entsprechen und/oder innerhalb der Sollbereiche liegen.

Zum Prüfen des Zerstäubers 1 wird der Zerstäuber 1 vorzugsweise in die Prüfvorrichtung 24 bzw. Halteeinrichtung 25 eingespannt bzw. von dieser aufgenommen. Besonders bevorzugt wird der Zerstäuber 1 in die Aufnahme 26 der Prüfvorrichtung 24 bzw. der Halteeinrichtung 25 eingesetzt, beispielsweise mittels eines nicht dargestellten Greifers oder sonstigem Manipulator (nicht dargestellt), und - vorzugsweise automatisch - eingespannt bzw. eingeklemmt.

Der Zerstäuber 1 weist vorzugsweise einen Energiespeicher, wie eine Feder, auf, vorzugsweise wobei der Energiespeicher vor der der Abgabe des Aerosols dienenden Betätigung des Zerstäubers 1 in einem Spannvorgang geladen bzw. gespannt wird.

Vorzugsweise wird der Zerstäuber 1 bereits im gespannten Zustand in die Prüfvorrichtung 24 bzw. Halteeinrichtung 25 eingesetzt bzw. von dieser aufgenommen. Es ist jedoch auch möglich, dass der (ungespannte) Zerstäuber 1 zunächst in die Prüfvorrichtung 24 bzw. Halteeinrichtung 25 eingesetzt bzw. von dieser aufgenommen wird und anschließend gespannt wird.

Insbesondere sind konstruktive Lösungen möglich, bei denen die Prüfvorrichtung 24 eine Spanneinrichtung (nicht dargestellt) aufweist, wobei der Zerstäuber 1 bzw. der Energiespeicher des Zerstäubers 1 mittels der Spanneinrichtung geladen bzw. gespannt wird.

Vorzugsweise wird der Zerstäuber 1 über bzw. unmittelbar vor die Messeinrichtung 27 bzw. den Emitter 28 platziert, insbesondere derart, dass der Behälter 3 bzw. Behälterboden 21 bestrahlt werden kann.

Vorzugsweise wird die Messeinrichtung 27 (anschließend) aktiviert bzw. eingeschaltet, vorzugsweise mittels der Steuereinrichtung 30 und/oder der Datenverarbeitungseinrichtung 31. Es ist jedoch auch möglich, dass die Messeinrichtung 27 permanent aktiviert bzw. eingeschaltet ist.

Vorzugsweise wird der Behälter 3 bzw. Behälterboden 21 - insbesondere mittels der Messeinrichtung 27 bzw. dem Emitter 28 - bestrahlt. Besonders bevorzugt wird die Strahlung vom bzw. am Behälter 3 bzw. Behälterboden 21 zumindest teilweise reflektiert, vorzugsweise zumindest teilweise in Richtung des Sensors 29.

Besonders bevorzugt wird die Position der am Behälter 3 bzw. Behälterboden 21 reflektierten Strahlung im Sensor 29 detektiert und/oder gespeichert.

Vorzugsweise wird der Abstand des Behälters 3 bzw. Behälterbodens 21 zu der Messeinrichtung 27 bzw. zu einer dem Zerstäuber 1 zugewandten Seite der Messeinrichtung 27 und/oder die Änderung des Abstandes des Behälters 3 bzw. Behälterbodens 21 zu der Messeinrichtung 27 bzw. zu einer dem Zerstäuber 1 zugewandten Seite der Messeinrichtung 27 bei der Abgabe des Fluids 2 gemessen, vorzugsweise mittels der Messeinrichtung 27. Vorzugsweise erfolgt die Messung dabei vor, während und nach der Abgabe des Fluids 2 bzw. kontinuierlich.

Vorzugsweise wird bei der Messung ein Messsignal 35 erzeugt und/oder an die Steuereinrichtung 30 und/oder die Datenverarbeitungseinrichtung 31 übermittelt.

Besonders bevorzugt werden kontinuierlich Messsignale 35 erzeugt und/oder übermittelt, vorzugsweise mit einer Frequenz von mehr als 1 kHz oder 2 kHz, besonders bevorzugt mehr als 5 kHz oder 10 kHz, insbesondere mehr als 20 kHz oder 50 kHz.

Das Messsignal 35 ist vorzugsweise ein Signal, das Informationen über den Abstand und/oder die Änderung des Abstands zwischen dem Behälter 3 bzw. Behälterboden 21 und der Messeinrichtung 27, den Winkel und/oder die Änderung des Winkels zwischen der vom Emitter 28 emittierten Strahlung und der am Behälter 3 bzw. Behälterboden 21 reflektierten Strahlung und/oder die Position und/oder Positionsänderung der vom Sensor 29 detektierten Strahlung aufweist.

Besonders bevorzugt weist das Messsignal 35 Informationen über den Hub Δs_{Hub}, die Geschwindigkeit v_{Hub} und/oder die Hubzeit Δt_{Hub} auf.

Ein Signal im Sinne der vorliegenden Erfindung ist vorzugsweise ein Mittel zur Informationsübertragung, eine (modulierte) Welle, insbesondere in einem Leiter, eine Bitsequenz, ein Paket im informationstechnischen Sinne o. dgl. Insbesondere ist ein Signal im Sinne der vorliegenden Erfindung über ein Übertragungsmedium bzw. mittels einer Datenverbindung übertragbar. Vorzugsweise sind einem Signal Informationen zugeordnet oder in dem Signal enthalten, die mittels des Signals übermittelbar sind.

Vorzugsweise wird der Zerstäuber 1 zur Abgabe des Fluids 2 betätigt, vorzugsweise mittels der Betätigungseinrichtung 32. Durch die Betätigung des Zerstäubers 1 wird das Fluid 2 zerstäubt bzw. das Aerosol 14 gebildet.

Optional wird die zum Spannen und/oder zur Betätigung des Zerstäubers 1 erforderliche Kraft gemessen, vorzugsweise mittels einer Messeinrichtung. Insbesondere wird gemessen, welche Kraft erforderlich ist, um das Sperrelement 8 für das Auslösen des Zerstäubers 1 zu bewegen bzw. um die Auslösetaste 8a zum Auslösen einzudrücken.

Vorzugsweise wird der Behälter 3 bzw. Behälterboden 21 bei der Entspannung der Antriebsfeder 7 bzw. Abgabe des Fluids 2 bzw. Bildung des Aerosols 14 und/oder nach der Betätigung des Zerstäubers 1 relativ zum Gehäuseteil 16 bewegt. Insbesondere wird der Behälter 3 bzw. Behälterboden 21 axial in Richtung der Austragsdüse 12 bewegt. Besonders bevorzugt führt der Behälter 3 bzw. Behälterboden 21 eine Hubbewegung bei der Abgabe des Fluids 2 aus.

Vorzugsweise werden/wird mittels der Messsignale 35 bzw. der Messwerte der Hub Δs_{Hub}, der Leerhub Δs_{Hub,1}, der Nutzhub Δs_{Hub,2}, die Hubzeit Δt_{Hub}, die Geschwindigkeit v_{Hub} und/oder die Beschleunigung des Behälters 3 ermittelt bzw. berechnet, vorzugsweise mittels der Datenverarbeitungseinrichtung 31.

Zusätzlich oder alternativ werden/wird mittels der Messsignale 35 bzw. der Messwerte die Druckverluste im Zerstäuber 1, die Strömungsgeschwindigkeit des Fluids 2 in der Austragsdüse 12 und/oder das Abgabevolumen bzw. die Abgabemasse des abgegebenen Fluids 2 bestimmt bzw. abgeschätzt, vorzugsweise mittels der Datenverarbeitungseinrichtung 31.

Vorzugsweise werden die Messsignale 35 bzw. Messwerte, insbesondere der Hub Δs_{Hub}, der Leerhub Δs_{Hub,1}, der Nutzhub Δs_{Hub,2}, die Zeit Δt_{Hub} und/oder die Geschwindigkeit v_{Hub} des Behälters 3 mit korrespondierenden Sollwerten, insbesondere einem Sollhub, einer Sollzeit und/oder einer Sollgeschwindigkeit, und/oder Grenzwerten, insbesondere einem maximalen und/oder minimalen Hub, einer maximalen und/oder minimalen Hubzeit und/oder einer maximalen und/oder minimalen Geschwindigkeit, verglichen.

Vorzugsweise sind die Sollwerte und/oder Grenzwerte in der Datenverarbeitungseinrichtung 31 und/oder einer (externen) Datenbank hinterlegt, insbesondere wobei die Datenverarbeitungseinrichtung 31 mit der Datenbank verbunden ist.

Vorzugsweise wird, vorzugsweise mittels der Aufnahmeeinrichtung 34 (lediglich in schematischer Weise in Fig. 3 angedeutet), das Fluid 2 bzw. Aerosol 14 bei der Abgabe bzw. das bei der Abgabe erzeugte Sprühbild optisch oder bildlich aufgenommen und/oder es erfolgt eine optische Messung des Sprühnebels bzw. Sprays / Aerosol 14.

Optional wird das Sprühbild an die Datenverarbeitungseinrichtung 31 übermittelt und/oder mit einem Referenzbild verglichen. Auf diese Weise können weitere Fehler des Zerstäubers 1, wie Fehler bei der Ausbildung der Aerosolwolke oder stark abgelenkte Sprühnebel, identifiziert, bestimmt und/oder abgeschätzt werden.

In einem weiteren, auch unabhängig realisierbaren Aspekt der vorliegenden Erfindung werden die Messsignale 35 bzw. Messwerte mit dem durch die Aufnahmeeinrichtung 34 aufgenommenen Sprühbild oder dem Ergebnis des Vergleichs des Sprühbilds mit dem Referenzbild kombiniert.

Insbesondere wird überprüft, ob die Bewegung des Behälters 3 zum Sprühbild korrespondiert bzw. ob eine bestimmte Bewegung des Behälters 3 zu einem erwarteten Sprühbild führt und/oder umgekehrt, ob bei einem bestimmten Sprühbild eine erwartete Bewegung,

Bewegungsgeschwindigkeit etc. des Behälters 3 bzw. des Behälterbodens 21 vorliegt. Auf diese Weise kann durch die Kombination der vorliegenden Informationen insbesondere ein Rückschluss auf die Funktionsfähigkeit des Druckerzeugers 5 und/oder der Austragsdüse 12 gezogen werden.

Besonders bevorzugt weist das System bzw. Prüfsystem 23 eine Sprayparametermesseinrichtung zur optischen Vermessung des Sprühnebel oder Sprays auf, insbesondere wobei die Aufnahmeeinrichtung 34 ein Teil der Sprayparametermesseinrichtung ist. Vorzugsweise ist die Sprayparametermesseinrichtung mit der Datenverarbeitungseinrichtung 31 verbunden und übermittelte Messdaten werden dort mit Referenzdaten verglichen.

Mittels der Sprayparametermesseinrichtung werden charakteristische Eigenschaften der vom Zerstäuber 1 abgegebenen Tropfenwolke bzw. des Sprays oder Sprühnebels vermessen. Vorzugsweise werden hierbei Messwerte generiert, die im System mit definierten Grenzwerten verglichen werden, insbesondere wobei diejenigen Zerstäuber 1 automatisch als fehlerhaft erkannt werden, für welche die ermittelten Messwerte außerhalb des durch die vordefinierten Grenzwerte bestimmten Wertebereichs liegen.

Gemäß einer bevorzugten Ausführung basiert das Funktionsprinzip der Sprayparametermesseinrichtung auf einem Lichtschnittverfahren. Hierbei wird vorzugsweise mit Laserlicht mindestens ein Lichtvorhang generiert, der den erzeugten Sprühnebel in einer definierten Ebene schneidet. Vorzugsweise verläuft die Ebene des Lichtvorhangs senkrecht zur erwarteten Hauptrichtung des Sprays und/oder senkrecht zu einer longitudinalen Hauptachse des Zerstäubers 1 und/oder senkrecht zu der Bewegungsrichtung des Behälters 3. Diese Schnittebene verläuft in einem definierten Abstand zur Düsenöffnung des Zerstäubers 1 bzw. wird in einem definiertem Abstand über dem Mundstück 13 des Zerstäubers 1 erzeugt. Beim Durchtritt des Sprühnebels bzw. Sprays durch den Lichtvorhang kommt es zur Streuung des Lichts an den Aerosoltropfen des Sprühnebels, wobei die Stärke der Lichtstreuung in direkter Abhängigkeit insbesondere von der Anzahl der Aerosoltropfen steht. Hierdurch entsteht in der Ebene des Lichtvorhangs ein Streulichtbild. Zur Erfassung des Streulichtbildes weist das System ein Kamerasystem und/oder eine Aufnahmeeinrichtung 34 auf. Vorzugsweise werden über einen vordefinierten Zeitraum und zu jeweils vorgegebenen Zeitpunkten (bezogen auf die Auslösung des Sprühnebels) mehrere Streulichtbilder erfasst. Vorzugsweise entspricht dieser Zeitraum der erwarteten Sprühzeit, bevorzugt 1 bis 1,5 Sekunden. Vorzugsweise wird mindestens viermal insgesamt ein Streulichtbild aufgenommen; besonders bevorzugt erfolgt die Erfassung der Streulichtbilder in regelmäßigen Abständen, z.B. alle 0,1 Sekunden.

Vorzugsweise wird bei der Auswertung der Streulichtbilder eine kegelförmige Gestalt des zu erwartenden Sprays zugrunde gelegt (Referenzdefinition). Bei der Lichtstreuung an so einer kegelförmigen Aerosolwolke nimmt die Intensität des gestreuten Lichts parabelförmig zur Mitte der Spraywolke zu. Entsprechend werden Auswerteparameter und zugehörige Grenzwerte definiert, die zu der erwarteten Spraykontur passen. Solche Auswerteparameter sind beispielsweise eine aufsummierte Streulichtintensität, Lage des Intensitätszentrums in Bezug zur Hauptachse und Intensitätsverteilung bzw. Varianz des Streulichts. Mit Hilfe einer solchen Sprayparametermesseinrichtung kann die Ausbildung des Sprühnebels überwacht werden, so dass Qualitätsmängel identifiziert werden, die beispielsweise auf verstopfte, falsch montierte oder gar beschädigte Düsen oder auf Ablagerungen im Düsenbereich zurückgehen.

Optional wird das Gewicht des Zerstäubers 1 ermittelt bzw. gemessen, vorzugsweise vor der Abgabe des Fluids 2, während der Abgabe des Fluids 2 und/oder nach der Abgabe des Fluids 2, insbesondere mittels einer Waage. Besonders bevorzugt sind solche Gewichtsmessungen jedoch Teil einer nachgeschalteten Laborprüfung an stichprobenartig ausgewählten Zerstäubern 1, welche insbesondere bereits an der Prüfvorrichtung 24 getestet wurden.

Durch die Berechnung der Differenz zwischen der gemessenen Masse des Zerstäubers 1 und/oder des Systems 23 bzw. der Prüfvorrichtung 24, insbesondere der Halteeinrichtung 25, vor der Abgabe des Fluids 2 und der gemessenen Masse des Zerstäubers 1 und/oder des Systems 23 bzw. der Prüfvorrichtung 24, insbesondere der Halteeinrichtung 25, nach der Abgabe des Fluids 2 kann die Masse des abgegebenen Fluids 2 bestimmt bzw. abgeschätzt werden.

Optional wird die gemessene bzw. berechnete Masse bzw. das Volumen des abgegebenen Fluids 2 bzw. Aerosols 14 mit Sollwerten bzw. Grenzwerten verglichen, vorzugsweise mittels der Datenverarbeitungseinrichtung 31.

### Bezugszeichenliste:

- 1: Zerstäuber
- 2: Fluid
- 3: Behälter
- 4: Fluidraum
- 5: Druckerzeuger
- 6: Halterung
- 7: Antriebsfeder
- 8: Sperrelement
- 8a: Auslösetaste
- 9: Förderrohr
- 10: Rückschlagventil
- 11: Druckkammer
- 12: Austragsdüse
- 13: Mundstück
- 14: Aerosol
- 15: Zuluftöffnung
- 16: Gehäuseteil
- 17: Innenteil
- 17a: oberer Teil (Innenteil)
- 17b: unterer Teil (Innenteil)
- 18: Gehäuseunterteil
- 19: Halteelement
- 20: Feder
- 21: Behälterboden
- 22: Anstechelement
- 23: System
- 24: Prüfvorrichtung
- 25: Halteeinrichtung
- 26: Aufnahme
- 27: Messeinrichtung
- 28: Emitter
- 29: Sensor
- 30: Steuereinrichtung
- 31: Datenverarbeitungseinrichtung
- 32: Betätigungseinrichtung
- 33: Gehäuse
- 34: Aufnahmeeinrichtung
- 35: Messsignal
- s: Strecke
- Δs_{Hub}: Hub
- Δs_{Hub,1}: Leerhub
- Δs_{Hub,2}: Nutzhub
- t: Zeit
- Δt_{Hub}: Hubzeit
- V_{Hub}: Hubgeschwindigkeit

## Patentansprüche

1. System (23) zum Prüfen der Funktionsfähigkeit eines Zerstäubers (1) zur Abgabe eines Fluids (2) als Aerosol (14),
wobei das System (23) den Zerstäuber (1) und eine automatisierte Prüfvorrichtung (24) aufweist,
wobei der Zerstäuber (1) einen vorzugsweise einsetzbaren und besonders bevorzugt auswechselbaren Behälter (3) mit dem Fluid (2) und ein Gehäuseteil (16) aufweist, wobei der Behälter (3) zur Abgabe des Fluids (2) relativ zum Gehäuseteil (16) bewegbar ist, und
wobei die automatisierte Prüfvorrichtung (24) eine Messeinrichtung (27) zum Messen der Bewegung des Behälters (3), insbesondere des Behälterbodens (21), bei der Abgabe des Fluids (2) aufweist,
wobei das System (23) eine Datenverarbeitungseinrichtung (31) aufweist, die mit der Messeinrichtung (27) verbunden oder verbindbar ist
**dadurch gekennzeichnet, dass**
die Datenverarbeitungseinrichtung (31) dazu ausgebildet ist, von der Messeinrichtung (27) ermittelte Messwerte auszuwerten und mit Sollwerten und/oder Grenzwerten zu vergleichen und den Zerstäuber (1) automatisch als fehlerhaft zu identifizieren, wenn die für ihn ermittelten Messwerte nicht den Sollwerten entsprechen und/oder außerhalb eines Bereichs liegen, der durch die Grenzwerte bestimmt ist, und
dass das System (23) eine Ausgabeeinrichtung aufweist, in welche der Zerstäuber (1) nach Identifizierung als fehlerhaft automatisch verschoben wird und/oder automatisch aussortiert wird.

2. System (23) nach Anspruch 1, **dadurch gekennzeichnet, dass** die Messeinrichtung (27) zum Messen der Geschwindigkeit (v_{Hub}) des Behälters (3), des Hubs (Δs_{Hub}) des Behälters (3) und/oder der Zeit (Δt_{Hub}) des Hubs (Δs_{Hub}) ausgebildet ist.

3. System (23) nach Anspruch 1 oder 2, **dadurch gekennzeichnet, dass** die automatisierte Prüfvorrichtung (24), zusätzlich zu der Messeinrichtung (27) eine Sprayparametermesseinrichtung zur Durchführung an Messungen an der vom Zerstäuber (1) erzeugten Aerosolwolke bzw. dem vom Zerstäuber (1) erzeugten Sprühnebel und/oder eine Aufnahmeeinrichtung (34) zur Erzeugung von Bildaufnahmen von der vom Zerstäuber (1) erzeugten Aerosolwolke bzw. dem vom Zerstäuber (1) erzeugten Sprühnebel aufweist.

4. System (23) nach Anspruch 3, **dadurch gekennzeichnet, dass** die Sprayparametermesseinrichtung und/oder die Aufnahmeeinrichtung mit der Datenverarbeitungseinrichtung (31) des Systems (23) verbunden ist und die Datenverarbeitungseinrichtung (31) dazu ausgebildet ist, von der Sprayparametermesseinrichtung ermittelte Messwerte bzw. von der Aufnahmeeinrichtung erzeugte Bilder auszuwerten und mit Sollwerten und/oder Grenzwerten bzw. Referenzbildern zu vergleichen und den Zerstäuber (1) automatisch als fehlerhaft zu identifizieren, wenn die für ihn ermittelten Messwerte bzw. Bilder nicht den Sollwerten bzw. Referenzbildern entsprechen und/oder außerhalb eines Bereichs liegen, der durch die Grenzwerte bzw. Referenzbilder bestimmt ist.

5. System (23) nach Anspruch 3 oder 4 **dadurch gekennzeichnet, dass** die Sprayparametermesseinrichtung ein Lichtvorhang erzeugt wird, der einen mittels des Zerstäubers (1) erzeugten Sprühnebel bzw. eine von dem Aerosol (14) gebildete Wolke in einer definierten Ebene schneidet.

6. System (23) nach einem der vorherigen Ansprüche, **dadurch gekennzeichnet, dass** das Fluid (2) im Behälter (3) eine reine Flüssigkeit, vorzugsweise Ethanol, ist.

7. System (23) nach einem der vorherigen Ansprüche, **dadurch gekennzeichnet, dass** die Prüfvorrichtung (24), eine Halteeinrichtung (25) zum Halten bzw. Einspannen des Zerstäubers (1), eine Steuereinrichtung (30), und/oder eine Betätigungseinrichtung (32) zum Betätigen des Zerstäubers (1) aufweist.

8. System (23) nach einem der voranstehenden Ansprüche, **dadurch gekennzeichnet, dass** die Messeinrichtung (27) als optische und/oder berührungslose Messeinrichtung ausgebildet ist und/oder einen Emitter (28), insbesondere einen Laser, und einen Sensor (29), insbesondere einen elektronischen Bildwandler, aufweist.

9. System (23) nach einem der voranstehenden Ansprüche, **dadurch gekennzeichnet, dass** die Messeinrichtung (27) ein Laser-Triangulations-Sensor-System beinhaltet oder durch ein Laser-Triangulations-Sensor-System gebildet ist.

10. Verfahren zum automatischen Prüfen der Funktionsfähigkeit eines Zerstäubers (1) zur Abgabe eines Fluids (2) als Aerosol (14),
wobei der Zerstäuber (1) einen vorzugsweise einsetzbaren und besonders bevorzugt auswechselbaren Behälter (3) mit dem Fluid (2) und ein Gehäuseteil (16) aufweist,
wobei der Behälter (3) zur Abgabe des Fluids (2) relativ zum Gehäuseteil (16) bewegt wird, und
wobei die Bewegung des Behälters (3), insbesondere des Behälterbodens (21), bei der Abgabe des Fluids (2) gemessen und/oder ausgewertet wird,
**dadurch gekennzeichnet, dass**
die bei der Bewegung des Behälter (3) ermittelten Messwerte ausgewertet und mit Sollwerten und/oder Grenzwerten verglichen werden und
der Zerstäuber (1) automatisch als fehlerhaft identifiziert wird, wenn die für ihn ermittelten Messwerte nicht den Sollwerten entsprechen und/oder außerhalb eines Bereichs liegen, der durch die Grenzwerte bestimmt ist, und
worauf der Zerstäuber (1) nach Identifizierung als fehlerhaft automatisch aussortiert wird.

11. Verfahren nach Anspruch 10, **dadurch gekennzeichnet, dass** die Geschwindigkeit (v_{Hub}), der Hub (Δs_{Hub}) und/oder die Zeit (Δt_{Hub}) des Hubs (Δs_{Hub}) - vorzugsweise mittels einer Messeinrichtung (27) - gemessen und/oder - vorzugsweise mittels einer Datenverarbeitungseinrichtung (31) - ausgewertet werden/wird und/oder mit Sollwerten, insbesondere einer Sollgeschwindigkeit, einem Sollhub und/oder einer Sollzeit, und/oder Grenzwerten, insbesondere einer maximalen und/oder minimalen Geschwindigkeit, einem maximalen und/oder minimalen Hub und/oder einer maximalen und/oder minimalen Zeit, verglichen werden/wird, vorzugsweise zur Ermittlung der Funktionsfähigkeit des Zerstäubers (1).

12. Verfahren nach einem nach einem der Ansprüche 10 oder 11, **dadurch gekennzeichnet, dass** der Zerstäuber (1) - vorzugsweise mittels einer Halteeinrichtung (25) - eingespannt bzw. eingeklemmt wird, und/oder dass der Zerstäuber (1) zur Abgabe des Fluids (2) - vorzugsweise mittels einer Betätigungseinrichtung (32) - betätigt wird.

13. Verfahren nach einem der Ansprüche 10 bis 12, **dadurch gekennzeichnet, dass** das Gewicht des abgegebenen Fluids (2) bzw. Aerosols (14) - vorzugsweise mittels einer Waage - gemessen wird und/oder dass das Fluid (2) bzw. Aerosol (14) bei der Abgabe - vorzugsweise mittels einer Sprayparametermesseinrichtung - optisch vermessen oder - vorzugsweise mittels einer Aufnahmeeinrichtung (34) -bildlich aufgenommen wird, vorzugsweise wobei ein ermittelte Gewichtswert oder ermittelte charakteristische Spraydaten mit einem Sollwert und/oder das aufgenommene Bild bzw. Sprühbild mit einem Referenzbild verglichen werden/wird.

14. Verfahren nach einem der Ansprüche 10 bis 13, **dadurch gekennzeichnet, dass** die Bewegung des Behälters (3) bzw. Behälterbodens (21), insbesondere die Geschwindigkeit (v_{Hub}), der Hub (Δs_{Hub}) und/oder die Zeit (Δt_{Hub}) der Bewegung bzw. des Hubs (Δs_{Hub}) des Behälters (3) bzw. des Behälterbodens (21), berührungslos, optisch und/oder durch Triangulation - vorzugsweise mittels einer Messeinrichtung (27) - gemessen werden/wird.

15. Verfahren nach einem der Ansprüche 10 bis 14, **dadurch gekennzeichnet, dass** der Behälterboden (21) bei bzw. zur Messung - vorzugsweise mittels einer Messeinrichtung (27) - bestrahlt wird und/oder insbesondere elektromagnetische Wellen am bzw. vom Behälterboden (21), insbesondere einer Reflexionsschicht des Behälters (3), reflektiert werden.

16. System nach Anspruch 1, wobei die automatisierte Prüfvorrichtung (24) zur Durchführung eines Verfahrens gemäß einem der Ansprüche 10 bis 15geeignet ist.

17. System nach Anspruch 16, wobei die Prüfvorrichtung (24) eine Sortierprüfvorrichtung ist, die geeignet für den Einsatz in einer 100%-Prüfung ist.

## Claims

1. System (23) for testing the proper functioning of an atomiser (1) for dispensing a fluid (2) in the form of an aerosol (14),
wherein the system (23) comprises the atomiser (1) and an automated test apparatus (24),
wherein the atomiser (1) comprises a preferably insertable and particularly preferably replaceable container (3) holding the fluid (2), and a housing part (16), the container (3) being movable relative to the housing part (16) in order to dispense the fluid (2), and
wherein the automated test apparatus (24) comprises a measuring device (27) for measuring the movement of the container (3), in particular of the container bottom (21), when the fluid (2) is dispensed,
wherein the system (23) comprises a data processing device (31) that is or can be connected to the measuring device (27),
**characterised in that**
the data processing device (31) is designed to analyse measured values detected by the measuring device (27) and to compare them with target values and/or limits and to automatically identify the atomiser (1) as being faulty when the measurements detected for it do not comply with the target values and/or are outside a range defined by the limits, and
**in that** the system (23) comprises an ejection device into which the atomiser (1) is automatically slid and/or automatically discarded once detected as being faulty.

2. System (23) according to claim 1, **characterised in that** the measuring device (27) is designed to measure the velocity (vₛₜᵣₒₖₑ) of the container (3), the stroke (Δsₛₜᵣₒₖₑ) of the container (3) and/or the duration (Δtₛₜᵣₒₖₑ) of the stroke (Δsₛₜᵣₒₖₑ).

3. System (23) according to either claim 1 or claim 2, **characterised in that** the automated test apparatus (24) comprises, in addition to the measuring device (27), a spray parameter measuring device for taking measurements on the aerosol cloud produced by the atomiser (1) or on the spray mist produced by the atomiser (1) and/or comprises a recording device (34) for generating images of the aerosol cloud produced by the atomiser (1) or of the spray mist produced by the atomiser (1).

4. System (23) according to claim 3, **characterised in that** the spray parameter measuring device and/or the recording device are connected to the data processing device (31) of the system (23) and the data processing device (31) is designed to analyse measured values detected by the spray parameter measuring device or images generated by the recording device and to compare them with target values and/or limits or reference images and to automatically identify the atomiser (1) as being faulty when the measured values or images detected for it do not comply with the target values or reference images and/or are outside a range defined by the limits or reference images.

5. System (23) according to either claim 3 or claim 4, **characterised in that** the spray parameter measuring device generates a light curtain that cuts through a defined plane in a spray mist produced by the atomiser (1) or a cloud formed by the aerosol (14).

6. System (23) according to any one of the preceding claims, **characterised in that** the fluid (2) in the container (3) is a pure liquid, preferably ethanol.

7. System (23) according to any one of the preceding claims, **characterised in that** the test apparatus (24), comprises a holding device (25) for holding or gripping the atomiser (1), a control device (30) and/or an actuation device (32) for actuating the atomiser (1).

8. System (23) according to any one of the preceding claims, **characterised in that** the measuring device (27) is designed as an optical and/or contactless measuring device and/or comprises an emitter (28), in particular a laser, and a sensor (29), in particular an electronic image converter.

9. System (23) according to any one of the preceding claims, **characterised in that** the measuring device (27) contains a laser triangulation sensor system or is formed by a laser triangulation sensor system.

10. Method for automated testing the proper functioning of an atomiser (1) for dispensing a fluid (2) in the form of an aerosol (14),
wherein the atomiser (1) comprises a preferably insertable and particularly preferably replaceable container (3) holding the fluid (2), and a housing part (16),
wherein the container (3) is moved relative to the housing part (16) in order to dispense the fluid (2), and
wherein the movement of the container (3), in particular of the container bottom (21), is measured and/or analysed when the fluid (2) is dispensed,
**characterised in that**
the measured values detected when the container (3) moves are analysed and compared with target values and/or limits, and
the atomiser (1) is automatically identified as being faulty when the measurements detected for it do not comply with the target values and/or are outside a range defined by the limits, and
upon which, after being identified as being faulty, the atomiser (1) is automatically discarded.

11. Method according to claim 10, **characterised in that** the velocity (vₛₜᵣₒₖₑ), the stroke (Δsₛₜᵣₒₖₑ) and/or the duration (Δtₛₜᵣₒₖₑ) of the stroke (Δsₛₜᵣₒₖₑ) are measured, preferably by means of a measuring device (27), and/or are analysed and/or compared, preferably by means of a data processing device (31), with target values, in particular a target velocity, a target stroke and/or a target duration, and/or with limits, in particular a maximum and/or minimum velocity, a maximum and/or minimum stroke and/or a maximum and/or minimum duration, preferably in order to determine whether the atomiser (1) is functioning properly.

12. Method according to either claim 10 or claim 11, **characterised in that** the atomiser (1) is gripped or clamped, preferably by means of a holding device (25), and/or **in that** the atomiser (1) is actuated, preferably by means of an actuation device (32), in order to dispense the fluid (2).

13. Method according to any one of claims 10 to 12, **characterised in that** the weight of the dispensed fluid (2) or of the aerosol (14) is measured, preferably by means of scales, and/or **in that** optical measurements are taken of the fluid (2) or aerosol (14) during the dispensing, preferably by means of a spray parameter measuring device, or images are taken thereof, preferably by means of a recording device (34), a detected weight value or detected characteristic spray data preferably being compared with a target value and/or the image or spray pattern recorded preferably being compared with a reference image.

14. Method according to any one of claims 10 to 13, **characterised in that** the movement of the container (3) or of the container bottom (21), in particular the velocity (vₛₜᵣₒₖₑ), the stroke (Δsₛₜᵣₒₖₑ) and/or the duration (Δtₛₜᵣₒₖₑ) of the movement or stroke (Δsₛₜᵣₒₖₑ) of the container (3) or container bottom (21), is/are measured contactlessly, optically and/or by triangulation, preferably by means of a measuring device (27).

15. Method according to any one of claims 10 to 14, **characterised in that**, during the measurement or for the purpose thereof, the container bottom (21) is irradiated, preferably by means of a measuring device (27), and/or in particular electromagnetic waves are reflected on or by the container bottom (21), in particular on or by a reflective coating of the container (3).

16. System according to claim 1, wherein the automated test apparatus (24) is suitable for carrying out a method according to any one of claims 10 to 15.

17. System according to claim 16, wherein the test apparatus (24) is a screening test apparatus which is suitable for use in a 100% inspection.

## Revendications

1. Système (23) de test du fonctionnement d'un pulvérisateur (1) pour la distribution d'un fluide (2) sous forme d'aérosol (14),
dans lequel le système (23) présente le pulvérisateur (1) et un dispositif de test automatisé (24),
dans lequel le pulvérisateur (1) présente un récipient (3) pouvant être de préférence inséré et de manière particulièrement préférée interchangeable avec le fluide (2) et une partie de boîtier (16), dans lequel le récipient (3) peut être déplacé par rapport à la partie de boîtier (16) pour distribuer le fluide (2), et
dans lequel le dispositif de test automatisé (24) présente un équipement de mesure (27) destiné à mesurer le déplacement du récipient (3), en particulier du fond du récipient (21), lors de la distribution du fluide (2),
dans lequel le système (23) présente un équipement de traitement de données (31), qui est connecté ou peut être connecté à l'équipement de mesure (27)
**caractérisé en ce que**
l'équipement de traitement de données (31) est réalisé pour évaluer des valeurs de mesure déterminées par l'équipement de mesure (27) et pour les comparer à des valeurs de consigne et/ou à des valeurs limites et pour identifier automatiquement le pulvérisateur (1) comme étant défectueux lorsque les valeurs de mesure déterminées pour celui-ci ne correspondent pas aux valeurs de consigne et/ou sont en dehors d'une plage qui est définie par les valeurs limites, et
que le système (23) présente un équipement de sortie dans lequel le pulvérisateur (1) est automatiquement déplacé et/ou est automatiquement évacué après avoir été identifié comme étant défectueux.

2. Système (23) selon la revendication 1, **caractérisé en ce que** l'équipement de mesure (27) est réalisé pour mesurer la vitesse (v_{Hub}) du récipient (3), la course (Δs_{Hub}) du récipient (3) et/ou le temps (Δt_{Hub}) de la course (Δs_{Hub}).

3. Système (23) selon la revendication 1 ou 2, **caractérisé en ce que** le dispositif de test automatisé (24) présente, en plus de l'équipement de mesure (27), un équipement de mesure de paramètre de pulvérisation destiné à être mis en œuvre sur des mesures sur le nuage d'aérosol généré par le pulvérisateur (1) ou sur le brouillard de pulvérisation généré par le pulvérisateur (1) et/ou un équipement d'enregistrement (34) destiné à générer des images du nuage d'aérosol généré par le pulvérisateur (1) ou du brouillard de pulvérisation généré par le pulvérisateur (1).

4. Système (23) selon la revendication 3, **caractérisé en ce que** l'équipement de mesure de paramètre de pulvérisation et/ou l'équipement d'enregistrement sont connectés à l'équipement de traitement de données (31) du système (23) et l'équipement de traitement de données (31) est réalisé pour évaluer des valeurs de mesure déterminées par l'équipement de mesure de paramètre de pulvérisation ou des images générées par l'équipement d'enregistrement et pour les comparer à des valeurs de consigne et/ou à des valeurs limites ou à des images de référence et pour identifier automatiquement le pulvérisateur (1) comme étant défectueux lorsque les valeurs de mesure ou les images déterminées pour lui ne correspondent pas aux valeurs de consigne ou aux images de référence et/ou se situent en dehors d'une plage qui est définie par les valeurs limites ou les images de référence.

5. Système (23) selon la revendication 3 ou 4, **caractérisé en ce que** l'équipement de mesure de paramètre de pulvérisation génère un rideau lumineux, qui coupe un brouillard de pulvérisation généré au moyen du pulvérisateur (1) ou un nuage formé par l'aérosol (14) dans un plan défini.

6. Système (23) selon l'une quelconque des revendications précédentes, **caractérisé en ce que** le fluide (2) dans le récipient (3) est un liquide pur, de préférence de l'éthanol.

7. Système (23) selon l'une quelconque des revendications précédentes, **caractérisé en ce que** le dispositif de test (24) présente un équipement de maintien (25) destiné à maintenir ou à enserrer le pulvérisateur (1), un équipement de commande (30), et/ou un équipement d'actionnement (32) destiné à actionner le pulvérisateur (1).

8. Système (23) selon l'une quelconque des revendications précédentes, **caractérisé en ce que** l'équipement de mesure (27) est réalisé en tant qu'équipement de mesure optique et/ou sans contact et/ou présente un émetteur (28), en particulier un laser, et un capteur (29), en particulier un convertisseur d'image électronique.

9. Système (23) selon l'une quelconque des revendications précédentes, **caractérisé en ce que** l'équipement de mesure (27) renferme un système de capteur de triangulation laser ou est formé par un système de capteur de triangulation laser.

10. Procédé de test automatique du fonctionnement d'un pulvérisateur (1) de distribution d'un fluide (2) sous forme d'aérosol (14),
dans lequel le pulvérisateur (1) présente un récipient (3) pouvant être de préférence inséré et de manière particulièrement préférée interchangeable avec le fluide (2) et une partie de boîtier (16),
dans lequel le récipient (3) est déplacé par rapport à la partie de boîtier (16) pour distribuer le fluide (2), et
dans lequel le déplacement du récipient (3), en particulier du fond du récipient (21), est mesuré et/ou évalué lors de la distribution du fluide (2),
**caractérisé en ce que**
les valeurs de mesure déterminées lors du déplacement du récipient (3) sont analysées et sont comparées à des valeurs de consigne et/ou à des valeurs limites et
le pulvérisateur (1) est automatiquement identifié comme étant défectueux lorsque les valeurs de mesure pour celui-ci ne correspondent pas aux valeurs de consigne et/ou se situent en dehors d'une plage qui est définie par les valeurs limites, et
le pulvérisateur (1) est alors automatiquement évacué après avoir été identifié comme étant défectueux.

11. Procédé selon la revendication 10, **caractérisé en ce que** la vitesse (v_{Hub}), la course (Δs_{Hub}) et/ou le temps (Δt_{Hub}) de la course (Δs_{Hub}) sont mesurés - de préférence au moyen d'un équipement de mesure (27) - et/ou sont évalués - de préférence au moyen d'un équipement de traitement de données (31) - et/ou sont comparés à des valeurs de consigne, en particulier à une vitesse de consigne, une course de consigne et/ou un temps de consigne, et/ou à des valeurs limites, en particulier à une vitesse maximale et/ou minimale, une course maximale et/ou minimale et/ou un temps maximal et/ou minimal, de préférence pour déterminer le fonctionnement du pulvérisateur (1).

12. Procédé selon l'une quelconque des revendications 10 ou 11, **caractérisé en ce que** le pulvérisateur (1) est enserré ou coincé - de préférence au moyen d'un équipement de maintien (25) -, et/ou que le pulvérisateur (1) est actionné - de préférence au moyen d'un équipement d'actionnement (32) - pour distribuer le fluide (2).

13. Procédé selon l'une quelconque des revendications 10 à 12, **caractérisé en ce que** le poids du fluide (2) ou de l'aérosol (14) distribué est mesuré - de préférence au moyen d'une balance - et/ou que le fluide (2) ou l'aérosol (14) est mesuré de manière optique lors de la distribution - de préférence au moyen d'un équipement de mesure de paramètres de pulvérisation - ou est photographié - de préférence au moyen d'un équipement d'enregistrement (34) -, de préférence dans lequel une valeur de poids déterminée ou des données caractéristiques de pulvérisation déterminées sont comparées à une valeur de consigne et/ou l'image ou la forme de pulvérisation enregistrée est comparée à une image de référence.

14. Procédé selon l'une quelconque des revendications 10 à 13, **caractérisé en ce que** le déplacement du récipient (3) ou du fond du récipient (21), en particulier la vitesse (v_{Hub}), la course (Δs_{Hub}) et/ou le temps (Δt_{Hub}) du déplacement ou de la course (Δs_{Hub}) du récipient (3) ou du fond du récipient (21) sont mesurés sans contact, de manière optique et/ou par triangulation - de préférence au moyen d'un équipement de mesure (27).

15. Procédé selon l'une quelconque des revendications 10 à 14, **caractérisé en ce que** le fond du récipient (21) est irradié lors de ou pour la mesure - de préférence au moyen d'un équipement de mesure (27) - et/ou en particulier des ondes électromagnétiques sont réfléchies sur ou par le fond du récipient (21), en particulier une couche réfléchissante du récipient (3).

16. Système selon la revendication 1, dans lequel le dispositif de test automatisé (24) est adapté pour mettre en œuvre un procédé selon l'une quelconque des revendications 10 à 15.

17. Système selon la revendication 16, dans lequel le dispositif de test (24) est un dispositif de test de tri qui est adapté pour l'utilisation dans un test à 100 %.
